# EUROPEAN PATENT SPECIFICATION

(11) **EP 3 302 716 B1**
(45) Date of publication and mention of the grant of the patent: **14.10.2020**
(21) Application number: 16729482.6
(22) Date of filing: 08.06.2016
(51) Int. Cl.: A61Q 17/04, G01N 33/50, A61Q 19/08, A61Q 19/00, C12Q 1/68

(54) **METHODS FOR IDENTIFYING CIRCADIAN RHYTHM-DEPENDENT COSMETIC AGENTS FOR SKIN CARE COMPOSITIONS**
VERFAHREN ZUR IDENTIFIZIERUNG VON BIORHYTHMUSABHÄNGIGEN KOSMETISCHEN MITTELN FÜR HAUTPFLEGEZUSAMMENSETZUNGEN
PROCÉDÉS D'IDENTIFICATION D'AGENTS COSMÉTIQUES DÉPENDANT DU RYTHME CIRCADIEN POUR DES COMPOSITIONS DE SOIN DE LA PEAU

(30) Priority: 08.06.2015 US 201562172498 P
(43) Date of publication of application: 11.04.2018
(73) Proprietor: The Procter & Gamble Company, Cincinnati, OH 45202 (US)
(72) Inventor: MULLINS, Lisa, Ann, Cincinnati, Ohio 45202 (US); TAMURA, Makio, Cincinnati, Ohio 45202 (US); OSBORNE, Rosemarie, Cincinnati, Ohio 45202 (US)
(74) Representative: P&G Patent Belgium UK
(86) International application number: PCT/US2016/036401
(87) International publication number: WO 2016/200905

(56) References cited:
- WO-A1-2010/079285
- JP-A- 2010 098 965
- US-A1- 2009 220 481
- US-A1- 2010 028 317
- US-A1- 2015 071 895
- GEYFMAN MIKHAIL ET AL: "Clock genes, hair growth and aging", AGING, NEW YORK, NY, US, vol. 2, no. 3, 1 March 2010 (2010-03-01), pages 122-128, XP002630052, ISSN: 0160-2721 [retrieved on 2010-03-18]

## Description

Skin is a complex, multi-layered and dynamic system that provides a protective covering defining the interactive boundary between an organism and the environment. It is the largest organ of the body and is vitally important to both our health and our self-image. The skin comprises three principal layers, the epidermis, the dermis, and a layer of subcutaneous fat. The majority of cells in the epidermis are keratinocytes that produce a family of proteins called keratins. The epidermis itself may be divided into multiple layers with the outermost layer referred to as the stratum corneum, and the innermost layer referred to as the basal layer. All epidermal cells originate from the basal layer and undergo a process known as differentiation as they gradually displace outward to the stratum corneum, where they fuse into squamous sheets and are eventually shed. In healthy, normal skin, the rate of production is about the same as the rate of shedding (desquamation). Fully mature keratinocytes function to protect the skin from UV light damage, and help effectuate immune response to environmental stimuli.

The dermis, which lies just beneath the epidermis, is composed largely of the protein collagen, which accounts for up to 75% of the weight of the dermis and is responsible for the resilience and elasticity of skin. Collagen bundles are held together by elastin fibers running through the dermis. Fibroblasts, which are the primary cells found in the dermis, function to synthesize collagen and the dermis ground substance, which is an extracellular matrix comprising glycoproteins and glycosaminoglycans that enmeshes fibrillar and cellular components of the dermis. Networks of tiny blood vessels run through "rete pegs" in the dermis, bringing nutrients, vitamins and oxygen to the epidermis via diffusion.

Beneath the dermis lies the hypodermis, which comprises subcutaneous fat that cushions the dermis from underlying tissues such as muscle and bones. The fat is contained in adipose cells embedded in a connective tissue matrix. This layer may also house the hair follicles when they are in the growing phase.

Thus, skin is a multilayered complex organ comprising a wide variety of cellular types and structures. Skin aging is likewise a complex multi-factorial process that results from unrepaired cellular and tissue damage leading to impaired functional capacity. The aging process in skin is the result of both intrinsic and extrinsic factors occurring over decades. Skin is subject to many of the same intrinsic aging processes as other organs, but is also exposed to solar radiation, pollution, cigarette smoke, and other extrinsic factors that can contribute to premature skin aging or photo-aging. There have been major advances in the understanding of the aging process with the identification of cellular pathways and genes associated with longevity and aging. However, as with aging in general, an integrated understanding of skin aging has not been developed.

Skin researchers have categorized age-inducing factors as either intrinsic or extrinsic, although these are interdependent, reflected for example by the fact that extrinsic factors may accelerate intrinsic aging. One example of the complex interplay of factors involves free radicals, which are both generated internally through normal metabolic processes and produced as a consequence of external factors, including UVR exposure. As a result of the age-associated decline in protective internal antioxidant mechanisms, free radicals can reach higher and sustained levels in cells and alter both proteins and DNA in skin. Levels of altered protein and DNA may accumulate causing damage, sometimes referred to as oxidative stress. In addition, ongoing accumulation of damage secondary to internally-generated free radicals combined with those generated from UVR and other external assaults (surfactants, allergens, and other irritants) can promote a chronic inflammatory state, which accelerates the aging process. For example, proteolytic enzymes may be produced, resulting in collagen degradation. In some instances, activated inflammatory cells resulting from elevations in circulating pro-inflammatory mediators (e.g., prostaglandins, cytokines, histamines) can produce reactive oxygen species that cause oxidative damage to nucleic acids, cellular proteins, and lipids. Accumulated damage caused by reactive oxygen species may stimulate a host of cytokine cascades that results in photo-aging and photo-carcinogenesis, all of which can be tied to the appearance of aging skin.

The changes caused by oxidative stress may compromise skin's elasticity, firmness and structure, contributing to areas of collapse and irregularity and ultimately manifesting as fine lines, wrinkles, and texture problems. There are many commercially available skin care products available to consumers that are directed to improving the health and/or physical appearance of skin. Many such products are directed to delaying, minimizing, or even eliminating changes typically associated with improving the appearance of aging skin. Such products typically advertise the use of one or more of cosmetic skin-care agents known for use in improving the health and/or appearance of skin. Accordingly, there remains a need to identify skin-care actives that can improve the appearance of aging skin.

Successful identification of new anti-aging cosmetic agents has proven to be difficult due to the multi-cellular, multi-factorial processes associated with skin aging. In addition, many desirable cosmetic agents may comprise a mixture of compounds with effects and interactions that may not be fully understood. An additional challenge for cosmetic formulators is that cosmetics must be safe for over-the-counter consumer use. Conventional *in vitro* studies of biological responses to potential cosmetic agents involve screening hundreds or even thousands of potential agents in various cell types before an agent that gives a desired result can be identified and moved into a next stage of testing. This problem may be further compounded when employing screening techniques such as connectivity mapping or other known gene response analysis techniques. Such studies can be hindered by the complex or weakly detectable gene responses typically induced and/or caused by cosmetic agents. Such weak responses arise, in part, due to the great number of genes and gene products involved, and cosmetic agents may affect multiple genes in multiple ways. Moreover, the degree of bioactivity of cosmetic agents may differ for each gene and be difficult to quantify.

Until now, skin studies typically have not taken into account the circadian rhythm of the skin and how the circadian rhythm of the skin affects the efficacy of the cosmetic agents. Patent application JP2010098965A discloses a screening assay for cosmetic material that takes into account the circadian rythm of the skin. Like other organs, the skin is subject to the influence of biologic rhythms. Biologic rhythms are physiologic changes occurring over time with a reproducible waveform. Cellular functions of the skin, including DNA repair, do not occur at random times with equal probability. Instead, these cellular activities are regulated by an endogenous clock having a circadian rhythm (24 hours). The body accepts environmental cues such as the presence and absence of daylight, to help synchronize the endogenous clocks of various cells and systems throughout the body. As such, the circadian cycle consists of light and dark cycles that typically coincide with the phases of solar day. For example, the light cycle may correspond to the hours of about 6:00 AM to 6:00 PM, and the dark cycle may correspond to the hours of about 6:00 PM to about 6:00 AM.

Circadian rhythms allow skin cells to anticipate changes in the environment that could potentially affect the cells, and adapt accordingly. In subject with good health and with properly functioning circadian rhythms, skin cells function in a synchronized manner and carry out their various functions at an optimal time. For example, the circadian rhythms of humans suggest that during the day, the skin promotes various protective functions with regard to the environment. The circadian rhythms of humans suggests that at night, the skin promotes cellular renewal and various metabolic synthesis processes. Although it is generally known that the efficacy of skin care treatments can be optimized in accordance time of day of administration and it is also been shown that both the circadian clock and biological rhythms are affected by the aging process, there are currently no methods available for identifying new anti-aging cosmetic agents that work in synchronization with the circadian rhythm of the skin to maximize anti-aging efficacy.

Thus, although many skin care agents are known, an ongoing need exists for improved, sensitive, and predicative screening methods to accurately identify new cosmetic agents that not only provide for the treatment of chronologically aged and/or photo-aged skin, but that also can more specifically target particular skin cell types and can work in synchronization with the circadian rhythm of the skin to maximize efficacy and optimize treatments. There is also a need to identify additional cosmetic agents that provide similar or improved benefits as compared to existing products but which are easier to formulate, produce, and/or market.

### SUMMARY

Accordingly, the present invention provides novel methods useful for the screening and generation of potential cosmetic agents that work in synchronization with the circadian rhythm of the skin for the treatment of aged skin. Through gene expression profiling and bioinformatics analysis, the present inventors have determined that it is possible to derive novel and unique gene signatures for use in developing novel screening methods for identifying cosmetic test agents as effective for providing a circadian rhythm-dependent, DNA repair benefit to human skin. These unique gene signatures may serve as indicators of previously unidentified pathways associated with DNA repair, and thus can provide opportunities for identifying new classes of cosmetic agents.

In some instances, the methods allow for the screening of cosmetic test agents that work in synchronization with the circadian rhythm of the skin, for example, the dark cycle of the skin's circadian cycle. These novel methods also allow for identification of new cosmetic agents that can be screened for their selective treatment of skin aging conditions and for the specific targeting of particular skin cell types, such as keratinocytes or fibroblasts. Thus, the invention provides methods uniquely suited for desired treatment targets. Additionally, these methods are particularly useful as they may serve as indicators of previously unidentified pathways associated with DNA repair, and thus can provide opportunities for identifying new classes of cosmetic agents.

Accordingly, Accordingly, a screening method for identifying a cosmetic test agent as effective for providing a circadian rhythm-dependent, DNA repair benefit to human skin is provided. The method comprises: (a) contacting a skin tissue sample with a cosmetic test agent; (b) generating a transcriptional profile for the skin tissue sample, wherein the transcriptional profile comprises data related to the transcription of at least two genes selected from *APITD1, ACTR5, AP5Z1, APEX1, APEX2, APLF, APTX, ATXN3, BCCIP, BRCA1, C11orf30, CDC14B, CHEK1, CUL4A, DCLRE1C, DTL, EPC2, EXO5, EYA3, FAM175A, FAN1, FANCC, FANCF, FANCG, FANCL, FIGNL1, GADD45A, GTF2H1, H2AFX, INTS3, KIAA0430, KIAA0101, KIN, MCM9, MDC1, MGME1, MORF4L2, MRE11A, MSH2, MUM1, NEIL1, NEIL3, NONO, NPM1, NSMCE1, OGG1, PARPBP, PIF1, PML, PMS1, PMS2, POLD1, POLD2, POLE, POLK, POLR2F, RAD50, RAD51B, RAD51C, RAD52, RBM14, RECQL5, RFC4, RFC5, RPA2, SFPQ, SFR1, SMARCA5, SMC4, SMG1, SWI5, TICRR, TP53BP1, TP73, UBE2T, UBE2W, USP28, USP47, WDR33, ZFYVE26;* (c) comparing the transcriptional profile for the skin tissue sample to a control transcriptional profile; and (d) identifying the cosmetic test agent as effective for providing a circadian rhythm-dependent, DNA repair benefit to human skin when the transcriptional profile for the skin tissue sample and the control transcriptional profile are concordant.

These and additional objects, embodiments, and aspects of the invention will become apparent by reference to the Figures and Detail Description below. The scope of the invention is defined by the appended claims.

### BRIEF DESCRIPTION OF THE FIGURES

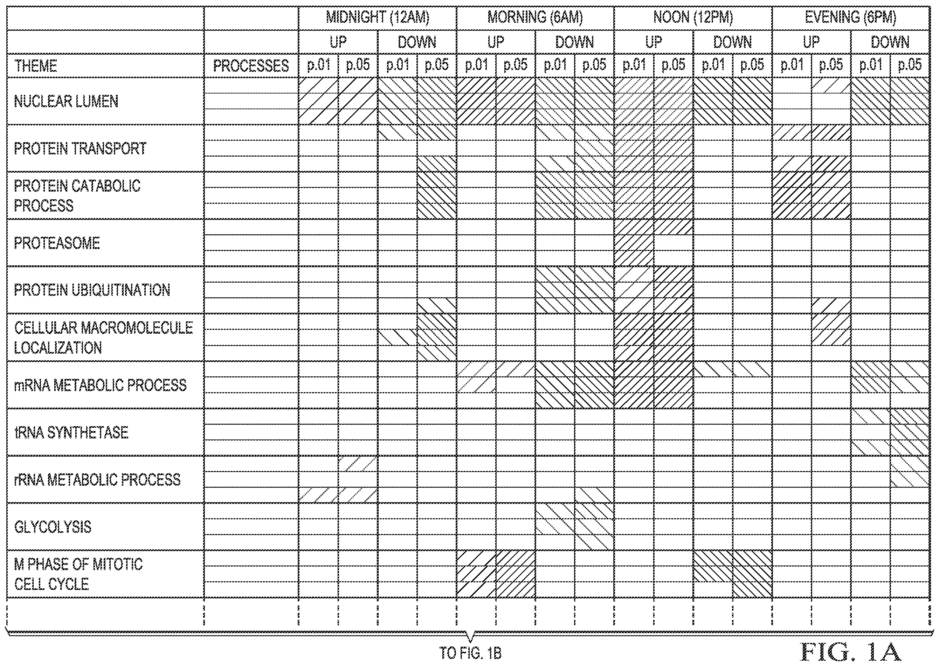

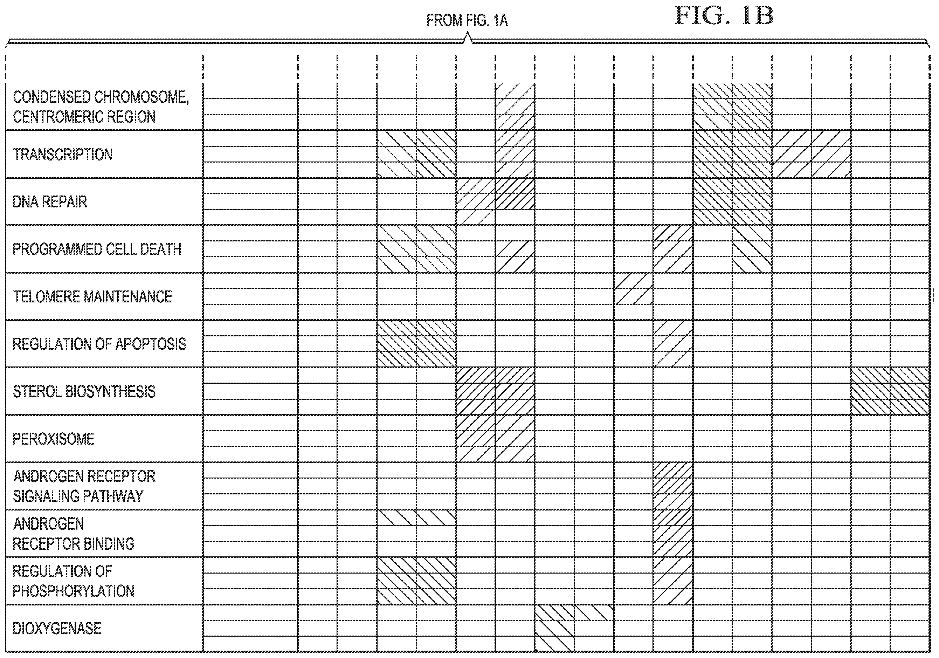

**FIGS. 1A** **and** **1B** illustrate that global gene expression profiles revealed rhythmic patterns of expression in a number of Gene Ontology categories, including DNA repair genes.

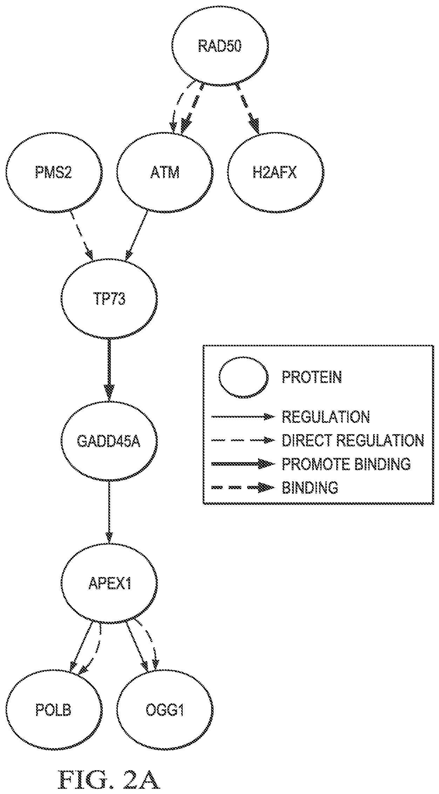

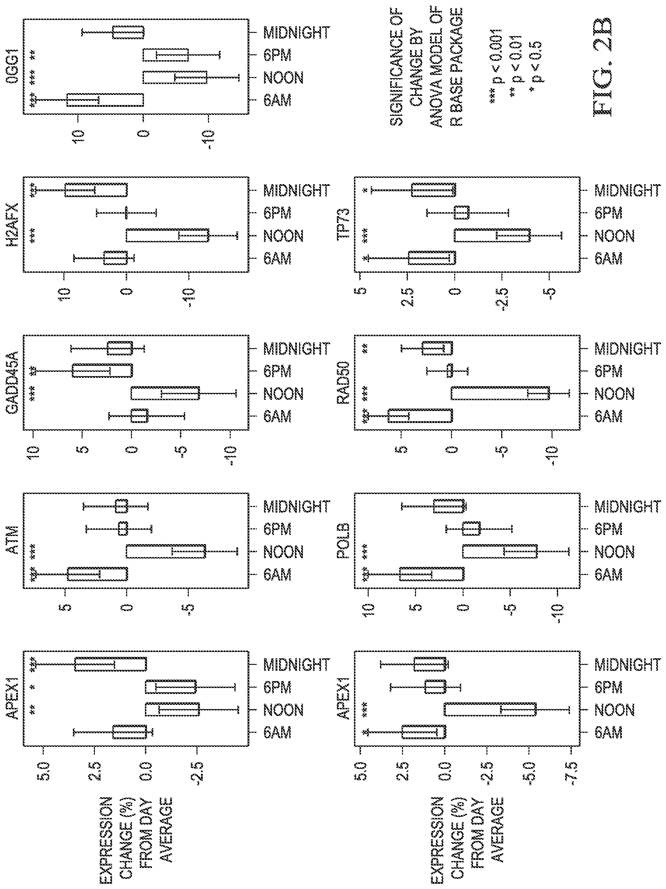

**FIG. 2** illustrates that several key genes controlling DNA repair and several metabolic processes displayed strong circadian rhythmicity. **FIG. 2A** illustrates the interaction network of representative DNA repair genes. **FIG. 2B** illustrates representative DNA repair genes expression changes.

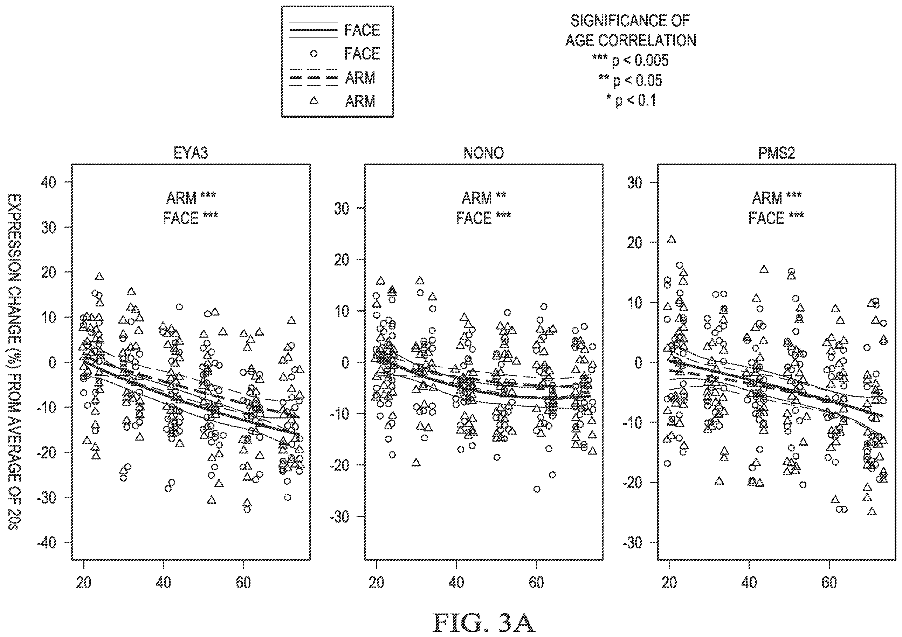

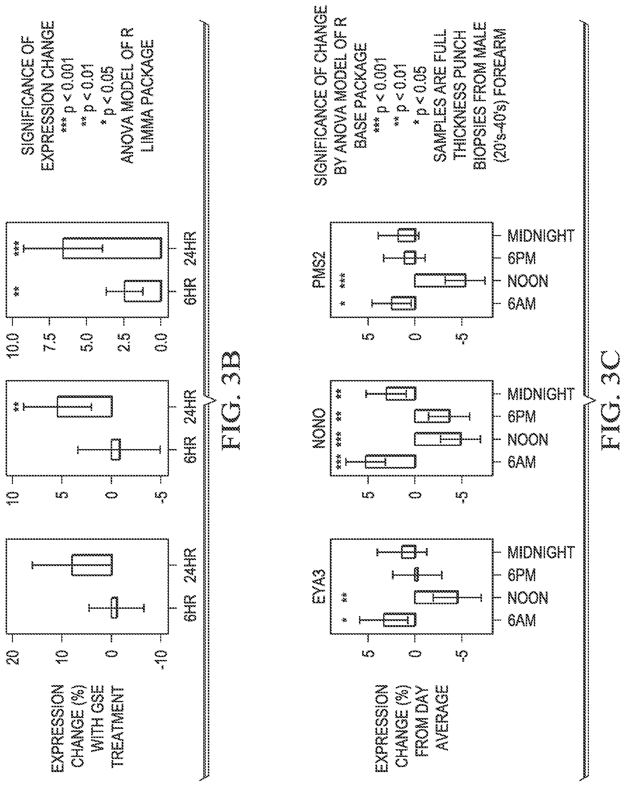

**FIG. 3** illustrates representative data for exemplary DNA repair genes in the epidermis. **FIG. 3A** illustrates the expression change (%) of exemplary genes in the epidermis due to aging (expression change % from average of 20s). **FIG. 3B** illustrates the expression change (%) of expression of representative DNA repair genes in keratinocytes in vitro with GSE treatment (0.01%). **FIG. 3C** illustrates the circadian pattern of representative DNA repair genes in the epidermis from full thickness punch biopsies.

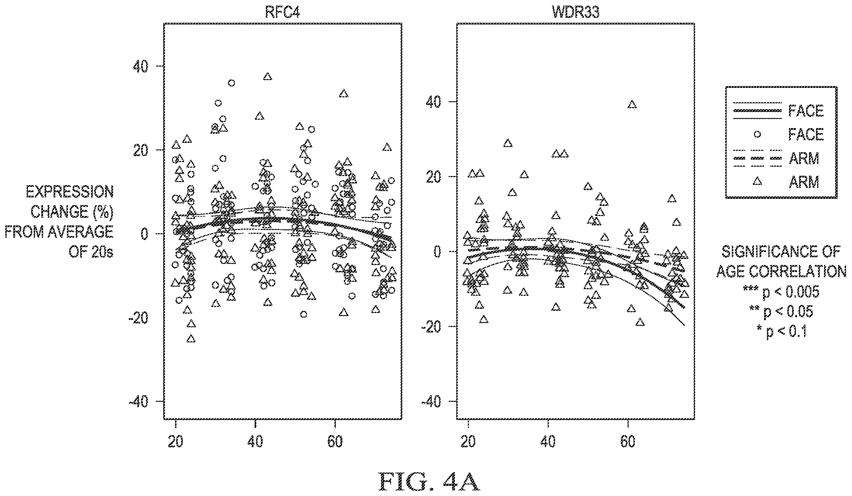

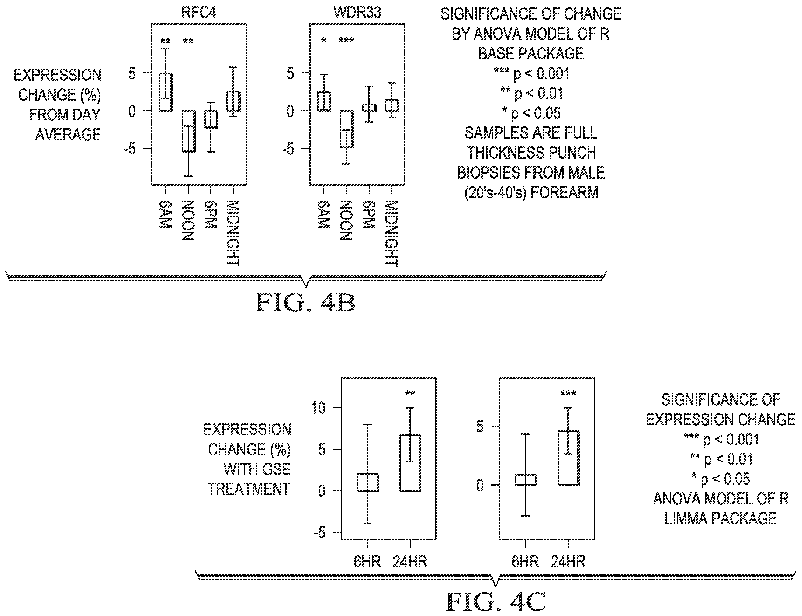

**FIG. 4** illustrates representative data for exemplary DNA repair genes in the dermis. **FIG. 4A** illustrates the expression change (%) of exemplary genes in the dermis due to aging (expression change % from average of 20s). **FIG. 4B** illustrates the expression change (%) of expression of representative DNA repair genes in fibroblasts in vitro with GSE treatment (0.01%). **FIG. 4C** illustrates the circadian pattern of representative DNA repair genes in the dermis from full thickness punch biopsies.

### DETAILED DESCRIPTION

Reference within the specification to "embodiment(s)" or the like means that a particular material, feature, structure and/or characteristic described in connection with the embodiment is included in at least one embodiment, optionally a number of embodiments, but it does not mean that all embodiments incorporate the material, feature, structure, and/or characteristic described. Furthermore, materials, features, structures and/or characteristics may be combined in any suitable manner across different embodiments, and materials, features, structures and/or characteristics may be omitted or substituted from what is described. Thus, embodiments and aspects described herein may comprise or be combinable with elements or components of other embodiments and/or aspects despite not being expressly exemplified in combination, unless otherwise stated or an incompatibility is stated.

All ingredient percentages are by weight of the corresponding composition, unless specifically stated otherwise. All ratios are weight ratios, unless specifically stated otherwise. All ranges are inclusive and combinable. The number of significant digits conveys neither a limitation on the indicated amounts nor on the accuracy of the measurements. All numerical amounts are understood to be modified by the word "about" unless otherwise specifically indicated. Unless otherwise indicated, all measurements are understood to be made at approximately 25 °C and at ambient conditions, where "ambient conditions" means conditions under about 1 atmosphere of pressure and at about 50% relative humidity. All numeric ranges are inclusive of narrower ranges; delineated upper and lower range limits are interchangeable to create further ranges not explicitly delineated.

The transcriptional profiles herein can comprise, consist essentially of, or consist of, data related to the genes in a subject gene signature (e.g., in the form of gene identifiers and direction of regulation) as well as other optional components described herein (e.g., metadata). As used herein, "consisting essentially of' means that a transcriptional profile includes data related to the transcription of only select genes from a subject gene signature or gene expression profile, but may also include additional data only if the additional data is not related to the transcription of genes not included in the subject gene signature, and which do not materially alter the basic and novel characteristics of the claimed compositions or methods. As used in the description and the appended claims, the singular forms "a," "an," and "the" are intended to include the plural forms as well, unless the context clearly indicates otherwise.

The genes disclosed herein correspond to their respective known sequences as of June 8, 2015.

### Definitions

"Circadian clock" means the endogenous cycle of about 24 hours that regulates the activities of a cell.

"Circadian rhythm-dependent benefit" means a benefit to keratinous tissue (including a DNA repair benefit) that works in synchronization with the circadian rhythm of human skin tissue.

"Circadian rhythm gene signature" means a gene signature derived from gene expression profiling of the circadian rhythm patterns of keratinous tissue.

"Cosmetic agent" or "cosmetic test agent" mean any substance, as well any component thereof, intended to be rubbed, poured, sprinkled, sprayed, introduced into, or otherwise applied to a mammalian body or any part thereof. Cosmetic agents may include substances that are Generally Recognized as Safe (GRAS) by the US Food and Drug Administration, food additives, and materials used in non-cosmetic consumer products including over-the-counter medications. In some embodiments, cosmetic agents may be incorporated in a cosmetic composition comprising a dermatologically acceptable carrier suitable for topical application to skin. Some non-limiting examples of cosmetic agents or cosmetically actionable materials can be found in: the PubChem database associated with the National Institutes of Health, USA; the Ingredient Database of the Personal Care Products Council; and the 2010 International Cosmetic Ingredient Dictionary and Handbook, 13th Edition, published by The Personal Care Products Council; the EU Cosmetic Ingredients and Substances list; the Japan Cosmetic Ingredients List; the Personal Care Products Council, the SkinDeep database; the FDA Approved Excipients List; the FDA OTC List; the Global New Products Database (GNPD); and from suppliers of cosmetic ingredients and botanicals.

"Dark cycle of the circadian clock" means the time period in which a subject sleeps. This is usually at night (absence of daylight). However, for certain subjects, the dark cycle can occur during the day, for example a person who works at night and sleeps during the day. Conversely, "light cycle" is the cycle of the circadian clock during which a subject is typically awake.

"Dermatologically acceptable" means that the compositions or components described are suitable for use in contact with human skin tissue.

"Gene expression profiling" and "gene expression profiling experiment" mean a measurement of the expression of multiple genes in a biological sample using any suitable profiling technology. For example, the mRNA expression of thousands of genes may be determined using microarray techniques. Other emerging technologies that may be used include RNA-Seq or whole transcriptome sequencing using NextGen sequencing techniques.

"Gene signature," means a rationally derived list, or plurality of lists, of genes representative of a skin tissue condition or response to a cosmetic agent. In specific contexts, the cosmetic agent may be a benchmark skin agent or a potential skin agent. Thus, the gene signature may serve as a proxy for a phenotype of interest for skin tissue. A gene signature may comprise genes whose expression, relative to a normal or control state, is increased (up-regulated), whose expression is decreased (down-regulated), and combinations thereof. Generally, a gene signature for a modified cellular phenotype may be described as a set of genes differentially expressed in the modified cellular phenotype over the cellular phenotype. A gene signature can be derived from various sources of data, including but not limited to, from in vitro testing, in vivo testing and combinations thereof. In some embodiments, a gene signature may comprise a first list representative of a plurality of up-regulated genes of the condition of interest and a second list representative of a plurality of down-regulated genes of the condition of interest.

"Intrinsic aging gene signature" means a gene signature derived from gene expression profiling of an intrinsic aging skin condition.

"Intrinsic aging skin condition" means a skin aging condition that derives, in whole or part, from chronological aging of the skin. "Intrinsically-aged" skin thus refers to skin that has been chronologically aged, and has been substantially protected from exposure to sunlight and/or ultraviolet light.

"Keratinous tissue," means keratin-containing layers disposed as the outermost protective covering of mammals which includes, but is not limited to, skin, hair, nails, cuticles, horns, claws, beaks, and hooves. With respect to skin, the term refers to one or all of the dermal, hypodermal, and epidermal layers, which includes, in part, keratinous tissue.

"Microarray" means any ordered array of nucleic acids, oligonucleotides, proteins, small molecules, large molecules, and/or combinations thereof on a substrate that enables gene expression profiling of a biological sample. Some non-limiting examples of microarrays are available from Affymetrix, Inc.; Agilent Technologies, Inc.; Ilumina, Inc.; GE Healthcare, Inc.; Applied Biosystems, Inc.; and Beckman Coulter, Inc.

"Photo-aging gene signature" means a gene signature derived from gene expression profiling of a photo-aging skin condition.

"Photo-aging skin condition" means a skin aging condition that derives, in whole or part, from exposure to sunlight and/or ultraviolet light (e.g., UVR, UVA, UVB, and/or UVC). "Photo-aged" skin thus refers to skin that has been exposed to sunlight and/or ultraviolet light.

"Safe and effective amount" means an amount of a compound or composition sufficient to significantly induce a positive benefit, preferably a positive skin or feel benefit, including independently or in combinations the benefits disclosed herein, but low enough to avoid serious side effects, i.e., to provide a reasonable benefit to risk ratio, within the scope of sound judgment of the skilled artisan.

"Skin" means the outermost protective covering of mammals that is composed of cells such as keratinocytes, fibroblasts and melanocytes. Skin includes an outer epidermal layer and an underlying dermal layer. Skin may also include hair and nails as well as other types of cells commonly associated with skin, such as, for example, myocytes, Merkel cells, Langerhans cells, macrophages, stem cells, sebocytes, nerve cells and adipocytes.

"Skin aging" means a human skin tissue condition resulting from the expression or repression of genes, environmental factors (e.g., sun exposure, UVA and/or UVB exposure, smoking), intrinsic factors (e.g. endogenous free radical production or cellular senescence) or interactions there between that produces one or more of fine lines and/or wrinkles, dry skin, inflamed skin, rough skin, sallow skin, telangectasia, sagging skin, enlarged pores, and combinations thereof.

"Skin-care" means regulating and/or improving a skin condition. Some non-limiting examples of "skin-care products" include skin creams, moisturizers, lotions, and body washes. "Skin-care composition" means a composition that regulates and/or improves skin condition.

"Topical application" means to apply or spread the compositions of the present invention onto the surface of the keratinous tissue.

Novel gene signatures have now been discovered which can be used to identify or evaluate the potential efficacy of a test agent for providing a skin-care benefit. In addition, novel methods useful for the screening and generation of potential cosmetic agents that work in synchronization with the circadian rhythm of the skin for the treatment of aged skin are provided herein. These novel methods exploit the newly discovered gene signatures to identify or evaluate the potential ability of a test agent to provide a targeted benefit to particular portion of skin or type of skin cell. Thus, the novel methods allow for the identification or evaluation of cosmetic agents that may provide maximize efficacy and allow for improved skin-care treatments. The genes of interest disclosed herein, and subsets thereof, may be used as gene panels to identify cosmetic agents that can provide a circadian-rhythm dependent DNA repair benefit to the epidermal and/or dermal layer of photo-aged and/or intrinsically-aged skin. Furthermore, these unique gene signatures may serve as indicators of previously unidentified pathways associated with DNA repair, and thus can provide opportunities for identifying new classes of cosmetic agents.

Gene expression may be detected and/or measured in a variety of ways. In certain embodiments, the method comprises measuring messenger ribonucleic acid ("mRNA") encoded by one or more genes of interest in a gene signature. Optionally, the method may include reverse transcribing mRNA encoded by one or more of the genes and measuring the corresponding complementary DNA ("cDNA"). Any suitable quantitative nucleic acid assay may be used herein. For example, conventional quantitative hybridization, Northern blot, and polymerase chain reaction procedures may be used for quantitatively measuring the amount of an mRNA transcript or cDNA in a biological sample. Optionally, the mRNA or cDNA may be amplified by polymerase chain reaction (PCR) prior to hybridization. The mRNA or cDNA sample is then examined by, e.g., hybridization with oligonucleotides specific for mRNAs or cDNAs encoded by one or more of the genes of the panel, optionally immobilized on a substrate (e.g., an array or microarray). Binding of the biomarker nucleic acid to oligonucleotide probes specific for the biomarker(s) allows identification and quantification of the biomarker. Suitable examples of methods of quantifying gene expression are disclosed in U.S. Publication No. 2012/0283112; U.S. application Ser. Nos. 13/851,858, 13/851,864, 13/851,873, and 13/851,886; and U.S. Ser. No. 13/966,418, filed by Mills, et al., on Aug. 15, 2012.

A non-limiting example of gene expression profiling involves a method of measuring gene expression and comparing the gene expression measurements to reference gene expression measurements (e.g., taken from a control sample comprises exposing test cells (e.g., keratinocytes and/or other skin cell) to a test agent, such as a cosmetic test agent. The test agent may be dissolved in a suitable carrier such as dimethyl sulfoxide (DMSO). Optionally, reference cells, which are typically the same type of cell as the test cells but which are only exposed to the carrier (i.e., no test agent), may be used as a control. After exposure to the test agent and/or control, mRNA is extracted from the test cells and reference cells. The mRNA extracted from the cells may, optionally, be reverse transcribed to cDNA and marked with fluorescent dye(s) (e.g., red and green if a two color microarray analysis is to be performed). In some instances, the cDNA samples may be prepped for a one color microarray analysis, and a plurality of replicates may be processed if desired. The cDNA samples may be co-hybridized to the microarray comprising a plurality of probes (e.g., tens, hundreds, or thousands of probes). In some embodiments, each probe on the microarray has a unique probe set identifier. The microarray is scanned by a scanner, which excites the dyes and measures the amount fluorescence. A computing device analyzes the raw images to determine the amount of cDNA present, which is representative of the expression levels of a gene. The scanner may incorporate the functionality of the computing device. Typically, gene expression data collected by the system may include: i) up-regulation of gene expression (e.g., greater binding of the test material (e.g., cDNA) to probes compared to reference material (e.g., cDNA)), ii) down-regulation of gene expression (e.g., reduced binding of the test material (e.g., cDNA) to probes than the test material (e.g., cDNA)), iii) non-fluctuating gene expression (e.g., similar binding of the test material (e.g., cDNA) to the probes compared to the reference material (e.g., cDNA)), and iv) no detectable signal or noise. The up- and down-regulated genes may be referred to as "differentially expressed." Differentially expressed genes may be further analyzed and/or grouped together (e.g., via known statistical methods) to identify genes that are representative of a particular skin condition or biological response to a cosmetic test agent.

The unique gene signatures developed for the methods herein are determined by bioinformatics analysis and comparison of gene expression profiles of skin aging conditions of interest, the gene expression profile of the circadian rhythm pattern of human skin tissue, and DNA repair genes. Gene signatures used for generating the unique gene signatures of interest may be generated, for example, from full thickness skin biopsies or other donor skin tissue (e.g., surgical waste) from skin, which exhibits a skin aging condition of interest, compared to a control. The gene signatures that can be used to provide the presently-disclosed novel screening method for identifying a test agent as effective for providing a circadian rhythm-dependent, DNA-repair benefit to human skin can involve analysis of RNA from full or partial human skin tissue samples or from simple cell types removed from such samples (such as through laser capture microdissection or physical cell or cell layer removal or other ways known in the art). Dermal and epidermal layers may be removed and analyzed separately or together. Additionally, cell lines can be used to generate such gene expression profiles and resulting signatures, such as keratinocyte cell lines or fibroblast cell lines. Profiles can be generated from such individual cells, layers, or from multiple cells, layers, parts (or in whole) of the human skin tissue sample or samples.

Two gene signature types for skin aging include an intrinsic aging gene signature and a photo-aging gene signature, which may be derived by comparing gene expression data from a full thickness skin biopsy from skin having the condition of interest and a control. Examples 2 and 3 below describe in greater detail non-limiting methods for deriving these gene signatures. Generally, for a photo-aging gene signature, biopsies may be taken from sun exposed skin (e.g., extensor forearm, cheek, forehead) and sun protected skin (e.g., buttocks, armpit, upper inner arm, upper inner thigh) of a plurality of older subjects. The subjects may vary in age, but one age range is between about 45 years of age and 70 years of age. A gene expression profiling analysis of the biopsy samples may be performed and one or more photo-aging gene signatures derived from a statistical analysis of the results. In some instances, a photo-aging gene signature may be derived by comparing a sun exposed site of an older individual (e.g., 45 to 80 y.o.) to a sun exposed site of a younger individual (e.g., 18 to 25 y.o.)

Generally, for an intrinsic aging gene signature, biopsies may be taken from sun protected sites (e.g., buttocks) of a plurality of older and younger subjects. The subjects may vary in age, but one age range is between about 45 years of age and 80 years of age for the older subjects and 18 years of age and 25 years of age for the younger subjects. A gene expression profiling analysis of the biopsy samples may be performed and one or more intrinsic aging gene signatures derived from a statistical analysis of the microarray results. In some embodiments, a photo-aging gene signature may be derived by comparing a sun protected site of an older individual (e.g., 45 to 80 y.o.) to a sun protected site of a younger individual (e.g., 18 to 25 y.o.)

Generally, for the gene signature of the circadian rhythm of human skin tissue, biopsies may be taken from healthy volunteers at four time points: 12 midnight, 6 am, 12 noon, and 6 pm. The subjects can vary in age, but one range is 20 to 74 years old. Another range is 20 to 40 years old. The subjects can be male or female. In some embodiments, the circadian rhythm gene signature may be derived by comparing the four time points. In other embodiments, the biopsies can be obtained from sun protected sites or may be obtained from sun exposed sites.

Gene signatures may also be derived from a gene expression profiling analysis of fibroblast and/or keratinocyte cells treated with a positive control skin agent to represent cellular perturbations leading to improvement in the skin tissue condition treated with that benchmark skin agent, said signature comprising a plurality of genes up-regulated and down-regulated by the benchmark skin agent in cells in vitro. As one illustrative example, microarray gene expression profile data where the positive control agent is the known skin anti-aging agent Golden Silk Extract ("GSE") (INCI name: Glycerin, Hydrolyzed Silk. CAS No. 56-81-5) may be analyzed using the present method. Thus, a list of genes strongly up-regulated and strongly down-regulated in response to challenge with GSE, or any other control agent or benchmark skin agent, can be derived. Said list of genes (a proxy for skin anti-aging) can be used in combination with the gene expression profiles of skin aging conditions of interest and the gene expression profile of the circadian rhythm pattern of human skin tissue to provide unique and new gene signature which can serve as query signatures to screen for skin anti-aging agents that will provide maximize efficacy and allow for the optimization of treatments. Furthermore, these unique gene signatures may serve as indicators of previously unidentified pathways associated with DNA repair, and thus can provide opportunities for identifying new classes of cosmetic agents. It is to be appreciated that numerous genes may be analyzed during gene profiling, but it is important to recognize that only the genes identified in the various gene signatures discussed below are selected for further analysis in the transcriptional profiles of the present method.

Non-limiting aspects and examples of various embodiments of skin screening methods will now be described. In certain embodiments, the methods allow for the screening of cosmetic agents that work in synchronization with the circadian rhythm of the skin. In more particular embodiments, the methods allow for the screening of cosmetic agents that work in synchronization with a dark cycle of the skin's circadian cycle. These novel methods also allow for identification or evaluation of cosmetic agents that can be screened for their selective treatment of skin aging conditions and for the specific targeting of particular skin cell types, such as keratinocytes or fibroblasts. Examples of skin aging conditions include intrinsic skin aging conditions, which are generally age-dependent, and photo-aged skin conditions, which are a form of skin aging where the skin is exposed to UV radiation.

In some instances, a screening method for identifying or evaluating the potential effectiveness of a cosmetic test agent for providing a circadian rhythm-dependent, DNA repair benefit to human skin is provided. For example, the method may comprise: (a) contacting a skin tissue sample (e.g., full thickness tissue sample or portion thereof) with a cosmetic test agent; (b) generating a transcriptional profile for the skin tissue sample ("test profile"), wherein the test profile comprises data related to the transcription of at least two genes selected from *APITD1*, *ACTR5*, *AP5Z1, APEX1, APEX2, APLF, APTX, ATXN3, BCCIP, BRCA1, C11orf30, CDC14B, CHEK1, CUL4A, DCLRE1C, DTL, EPC2, EXO5, EYA3, FAM175A, FAN1, FANCC, FANCF, FANCG, FANCL, FIGNL1, GADD45A, GTF2H1, H2AFX, INTS3, KIAA0430, KIAA0101, KIN, MCM9, MDC1, MGME1, MORF4L2, MRE11A, MSH2, MUM1, NEIL1, NEIL3, NONO, NPM1, NSMCE1, OGG1, PARPBP, PIF1, PML, PMS1, PMS2, POLD1, POLD2, POLE, POLK, POLR2F, RAD50, RAD51B, RAD51C, RAD52, RBM14, RECQL5, RFC4, RFC5, RPA2, SFPQ, SFR1, SMARCA5, SMC4, SMG1, SWI5, TICRR, TP53BP1, TP73, UBE2T, UBE2W, USP28, USP47, WDR33,* and *ZFYVE26;* (c) comparing the transcriptional profile for the skin tissue sample to a control transcriptional profile; and (d) identifying the cosmetic test agent as having potential effectiveness for providing a circadian rhythm-dependent, DNA repair benefit to human skin when the transcriptional profile for the skin tissue sample, relative to the control transcriptional profile, corresponds to regulation (i.e., upregulation or downregulation) of the at least two genes in a direction indicative of a circadian rhythm-dependent, DNA repair benefit to human skin. For example, if the test transcriptional profile and a positive control transcriptional profile (i.e., a control transcriptional profile generated by contacting a test sample with a positive control) are concordant, meaning that expression of the genes in the respective transcriptional profiles is regulated in the same direction, then the test agent can be identified as exhibiting potential effectiveness for providing said benefit.

In the foregoing example, the test profile may consist essentially of data related to the transcription of at least two genes selected from gene signature. That is, the transcriptional profile does not include data related to the transcription of other genes not listed.

While the above example and some of the examples that follow utilize a positive control, it is to be appreciated that embodiments wherein a transcriptional profile is generated using a vehicle control or a negative control are also contemplated herein, and it is within the skill of the ordinary artisan to determine whether a test agent has potential effectiveness for providing a circadian rhythm-dependent, DNA repair benefit to human skin by comparing a test transcriptional profile to a control transcriptional profile generated with a vehicle or negative control. For example, if the test transcriptional profile and a negative control transcriptional profile (i.e., a control transcriptional profile generated by contacting a test sample with a negative control) are discordant, meaning that expression of the genes in the respective transcriptional profiles is regulated in generally opposite directions, then the test agent can be identified as having potential effectiveness for providing a circadian rhythm-dependent, DNA repair benefit to human skin

In some instances, the transcriptional profile for the skin tissue sample contacted with a cosmetic test agent (a first skin tissue sample) can be compared to a second skin tissue sample contacted with a control agent. In such an embodiment, the cosmetic test agent can be identified as effective for providing a circadian rhythm-dependent, DNA repair benefit to human skin when a comparison of the transcriptional profiles of the first and second skin tissue samples indicates regulation of at least two genes selected from one of the gene signatures above in a direction corresponding to a circadian rhythm-dependent, DNA repair benefit to human skin..

In some instances, it may be desirable to select a gene signature wherein the transcription profile for the human skin tissue sample comprises data related to the transcription of at least two genes selected from *CUL4A, EYA3, FAN1, NONO, OGG1, PMS2, RFC4, WDR33, KIN, NPM1, RAD50, APEX1, BCCIP, DTL, MGME1, NSMCE1, PIF1, POLK, RAD51C, SFR1, UBE2W, ZFYVE26, CHEK1, GADD45A, MSH2, PARPBP, RFC5, SMC4, UBE2T, APITD1, CHEK1, KIAA0101, ATXN3,* and *BRCA1.* In a particularly suitable example, the transcriptional profile may comprise data related to the transcription of at least two genes selected from *H2AFX, NSMCE1, RAD51C, UBE2T,* and *WDR33.*

In some instances, it may be desirable to identify or evaluate the potential effectiveness of a cosmetic test agent for providing a circadian rhythm-dependent, DNA repair benefit to intrinsically-aged human skin. For example, the method may comprise: (a) contacting a skin tissue sample with a cosmetic test agent; (b) generating a transcriptional profile for the skin tissue sample, wherein the transcriptional profile comprises data related to the transcription of at least two genes selected from *APITD1, APEX1, APTX, ATXN3, BRCA1, C11orf30, CDC14B, CHEK1, DCLRE1C, DTL, EPC2, EXO5, EYA3, FAM175A, FANCF, GADD45A, GTF2H1, H2AFX, KIAA0101, KIN, MORF4L2, NEIL3, NPM1, NSMCE1, OGG1, PARPBP, PIF1, PML, POLD2, RAD50, RAD51B, RAD51C, RFC4, SMARCA5, SMC4, TP53BP1, UBE2T, UBE2W,* and *WDR33;* (c) comparing the transcriptional profile for the skin tissue sample to a control transcriptional profile; and (d) identifying the cosmetic test agent as exhibiting potential effectiveness for providing a circadian rhythm-dependent, DNA repair benefit to intrinsically-aged human skin when the transcriptional profile for the skin tissue sample, relative to the control transcriptional profile, corresponds to regulation of the genes in a direction indicative of said benefit.. In this example, it may be desirable to generate a transcriptional profile comprising data related the transcription of at least two genes selected from a subset of the foregoing list of genes, such as *APTX, C11orf30, FANCF, H2AFX, KIAA0101, KIN, NPM1, NSMCE1, RAD50, RAD51C, SMARCA5, SMC4, UBE2T,* and *WDR33.* In this example, it may be more desirable to use 0.1% GSE as a positive control and generate a transcriptional profile for the skin sample comprising data related to the transcription of at least two genes selected from *APITD1, CHEK1, KIAA0101, NSMCE1, PIF1, RAD50, RAD51C, ATXN3, BRCA1, DTL, PARPBP, RFC4, SMC4, UBE2T,* and *WDR33.* In this example, it may be even more desirable to use 0.01% GSE as a positive control and generate a transcriptional profile for the skin sample comprising data related to the transcription of at least two genes selected from *EYA3, KIN, NPM1, RAD50, RFC4,* and WRD33.In some instances, it may be desirable to identify or evaluate the potential effectiveness of a cosmetic test agent for providing a circadian rhythm-dependent, DNA repair benefit to photo-aged human skin. For example, the method may comprise: (a) contacting a skin tissue sample with a cosmetic test agent; (b) generating a transcriptional profile for the skin tissue sample, wherein the transcriptional profile comprises data related to the transcription of at least two genes selected from *ACTR5, AP5Z1, APEX1, APEX2, APLF, APTX, ATXN3, BCCIP, BRCA1, C11orf30, CDC14B, CHEK1, CUL4A, DTL, EPC2, EYA3, FAM175A, FAN1, FANCC, FANCF, FANCG, FANCL, FIGNL1, GADD45A, GTF2H1, H2AFX, INTS3, KIAA0430, KIAA0101, KIN, MCM9, MDC1, MGME1, MORF4L2, MRE11A, MSH2, MUM1, NEIL1, NEIL3, NONO, NPM1, NSMCE1, OGG1, PARPBP, PIF1, PMS1, PMS2, POLD1, POLD2, POLE, POLK, POLR2F, RAD50, RAD51B, RAD51C, RAD52, RBM14, RECQL5, RFC4, RFC5, RPA2, SFPQ, SFR1, SMARCA5, SMC4, SMG1, SWI5, TICRR, TP53BP1, TP73, UBE2T, UBE2W, USP28, USP47, WDR33,* and *ZFYVE26;* (c) comparing the transcriptional profile for the skin tissue sample to a control transcriptional profile; and (d) identifying the cosmetic test agent as exhibiting potential effectiveness for providing a circadian rhythm-dependent, DNA repair benefit to photo-aged human skin when the transcriptional profile for the skin tissue sample, relative to the control transcriptional profile, corresponds to regulation of the genes in a direction indicative of said benefit. In this example, it may also be desirable to use 0.01% GSE as a positive control and generate a transcriptional profile comprising data related the transcription of at least two genes selected from *APEX1, BCCIP, CUL4A, DTL, EYA3, FAN1, MGME1, NONO, NSMCE1, OGG1, PIF1, PMS2, POLK, RAD50, RAD51C, SFR1, UBE2W, ZFYVE26, CHEK1, GADD45A, MSH2, PARPBP, RFC4, RFC5, RPA1, SMC4, UBE2T,* and *WDR33.* In this example, it may be particularly desirable to use 0.1% GSE as a positive control and generate a transcriptional profile comprising data related the transcription of at least two genes selected from *APEX1, CHEK1, DTL, EYA3, FAN1, H2AFX, MORF4L2, MSH2, NONO, NSMCE1, POLD2, RAD51C, RFC4, SWI5, TP53BP1, UBE2T, UBE2W,* and *WDR33.*

In some instances, it may be desirable to identify or evaluate the potential effectiveness of a cosmetic test agent for providing a circadian rhythm-dependent, DNA repair benefit to the epidermis and/or the cells found therein. For example, a full thickness skin sample may be separated into epidermis and dermis layer, such that that the epidermis may be analyzed separately. Additionally or alternatively, keratinocytes may be cultured and tested according to the present method.. In such an embodiment, the method may comprise: (a) contacting a sample of keratinocytes with a cosmetic test agent; (b) generating a transcriptional profile for the sample of keratinocytes, wherein the transcriptional profile comprises data related to the transcription of at least two genes selected from *APTX, C11orf30, CDC14B, CHEK1, EPC2, EYA3, FANCF, GTF2H1, H2AFX, KIN, NEIL3, NSMCE1, PIF1, RAD50, RAD51C, SMARCA5, UBE2T,* and *WDR33;* (c) comparing the transcriptional profile for the sample of keratinocytes to a control transcriptional profile; and (d) identifying the test agent as exhibiting potential effectiveness for providing a circadian rhythm-dependent, DNA repair benefit to the epidermal layer of human skin when the transcriptional profile for the keratinocyte sample, relative to the control transcriptional profile, corresponds to regulation of the genes in a direction indicative of said benefit.

In some instances, it may be desirable to identify or evaluate the potential effectiveness of a cosmetic test agent for providing a circadian rhythm-dependent, DNA repair benefit to an epidermal layer of intrinsically-aged human skin. For example, the method may comprise: (a) contacting a test sample (e.g., keratinocytes or epidermal tissue) with a cosmetic test agent; (b) generating a transcriptional profile for the test sample, wherein the transcriptional profile comprises data related to the transcription of at least two genes selected from *APITD1, APTX, C11orf30, CDC14B, CHEK1, DCLRE1C, EPC2, EXO5, EYA3, FANCF, GADD45A, GTF2H1, H2AFX, KIAA0101, KIN, NEIL3, NPM1, NSMCE1, PIF1, RAD50, RAD51B, RAD51C, SMARCA5, SMC4, UBE2T,* and *WDR33;* (c) comparing the transcriptional profile for the test sample to a control transcriptional profile; and (d) identifying the cosmetic test agent as exhibiting potential effectiveness for providing a circadian rhythm-dependent, DNA repair benefit to the epidermal layer of intrinsically-aged human skin when the transcriptional profile for the test sample, relative to the control transcriptional profile, corresponds to regulation of the genes in a direction indicative of said benefit. In this example, it may be desirable to use 0.1% GSE as a positive control and generate a transcriptional profile for the test sample comprising data related to the transcription of at least two genes selected from *APITD1, CHEK1, KIAA0101, NSMCE1, PIF1, RAD50,* and *RAD51C.* In this example, it may be more desirable to use 0.01% GSE as a positive control and generate a transcriptional profile for the test sample comprising data related to the transcription of *EYA3.*

In some instances, it may be desirable to identify or evaluate the potential effectiveness of a cosmetic test agent for providing a circadian rhythm-dependent, DNA repair benefit to an epidermal layer of photo-aged human skin. For example, the method may comprise: (a) contacting a test sample (e.g., keratinocytes or epidermal tissue) with a cosmetic test agent; (b) generating a transcriptional profile for the test sample, wherein the transcriptional profile comprises data related to the transcription of at least two genes selected from *ACTR5, AP5Z1, APEX1, APLF, ATXN3, BCCIP, BRCA1, C11orf30, CDC14B, CHEK1, CUL4A, DTL, EPC2, EYA3, FAM175A, FAN1, FANCF, FANCG, FANCL, FIGNL1, GTF2H1, H2AFX, INTS3, KIAA0430, KIN, MCM9, MDC1, MGME1, MORF4L2, MRE11A, MSH2, MUM1, NEIL1, NEIL3, NONO, NSMCE1, OGG1, PIF1, PMS1, PMS2, POLD1, POLD2, POLE, POLK, RAD50, RAD51C, RAD52, RBM14, RECQL5, RFC4, SFR1, SMARCA5, SMG1, SWI5, TICRR, TP53BP1, UBE2T, UBE2W, USP47, WDR33,* and *ZFYVE26;* (c) comparing the transcriptional profile for the test sample to a control transcriptional profile; and (d) identifying the cosmetic test agent as exhibiting potential effectiveness for providing a circadian rhythm-dependent, DNA repair benefit to the epidermal layer of photo-aged human skin when the transcriptional profile for the test sample, relative to the control transcriptional profile, corresponds to regulation of the genes in a direction indicative of said benefit. In this example, it may be desirable to use a positive control (e.g., GSE) and generate a transcriptional profile for the test sample comprising data related to the transcription of at least two genes selected from *APEX1, BCCIP, CUL4A, DTL, EYA3, FAN1, MGME1, NONO, NSMCE1, OGG1, PIF1, PMS2, POLK, RAD50, RAD51C, SRF1, UBE2W,* and *ZFYVE26.* In this example, it may be more desirable to use 0.1% GSE as a positive control and generate a transcriptional profile for the test sample comprising data related to the transcription of at least two genes selected from *APEX1, BCCIP, CUL4A, DTL, FAN1, MGME1, NSMCE1, PIF1, PMS2, POLK, RAD50, RAD51C, SFR1, UBE2W,* and *ZFYVE26.* In this example, it may be even more desirable to use 0.01% GSE as a positive control and generate a transcriptional profile for the test sample comprising data related to the transcription of at least two genes selected from *CUL4A, EYA3, FAN1, NONO, OGG1,* and *PMS2.*

In some instances, the transcriptional profile for a sample of keratinocytes contacted with a cosmetic test agent (a first sample of keratinocytes) can be compared to a second sample of keratinocytes contacted with a control agent. In such an embodiment, the cosmetic test agent can be identified as exhibiting potential effectiveness for providing a circadian rhythm-dependent, DNA repair benefit to the epidermal layer of intrinsically-aged human skin when the transcriptional profiles of the first and second samples of keratinocytes, relative to one another, correspond to regulation of at least two genes of interest in a direction indicative of said benefit. The disclosed genes of interest in the above gene signatures may be suitable for use in this example. Additionally, the foregoing disclosed genes of interest may serve as indicators of previously unidentified pathways associated with DNA repair, and thus can provide opportunities for identifying new classes of cosmetic agents.

In some instances, the present screening method may be used to identify or evaluate the potential effectiveness of a cosmetic test agent for providing a circadian rhythm-dependent, DNA repair benefit to a dermal layer of human skin. For example, the method may comprise: (a) contacting a test sample (e.g., fibroblasts or dermal tissue) with a cosmetic test agent; (b) generating a transcriptional profile for the test sample, wherein the transcriptional profile comprises data related to the transcription of at least two genes selected from *APEX1, APTX, ATXN3, BRCA1, C11orf30, DTL, FAM175A, FANCF, H2AFX, KIAA0101, KIN, MORF4L2, NPM1, NSMCE1, OGG1, PARPBP, PML, POLD2, RAD50, RAD51C, RFC4, SMARCA5, SMC4, TP53BP1, UBE2T, UBE2W,* and *WDR33;* (c) comparing the transcriptional profile for the test sample to a control transcriptional profile; and (d) identifying the cosmetic test agent as exhibiting potential effectiveness for providing a circadian rhythm-dependent, DNA repair benefit to the dermal layer of human skin when the transcriptional profile for the test sample, relative to the control transcriptional profile, corresponds to regulation of the genes in a direction indicative of said benefit. In this example it may be desirable to generate a transcriptional profile for the test sample comprising data related to the transcription of at least two genes selected from *APEX1, APTX, DTL, H2AFX, KIAA0101, MORF4L2, NSMCE1, PARPBP, POLD2, RAD51C, RFC4, SMC4, TP53BP1, UBE2T, UBE2W,* and *WDR33.*

In some instances, it may be desirable to identify or evaluate the potential effectiveness of a cosmetic test agent for providing a circadian rhythm-dependent, DNA repair benefit to a dermal layer of intrinsically-aged human skin. For example, the method may comprise: (a) contacting a sample of fibroblasts with a cosmetic test agent; (b) generating a transcriptional profile for the sample of fibroblasts, wherein the transcriptional profile comprises data related to the transcription of at least two genes selected from *APEX1, APTX, ATXN3, BRCA1, C11orf30, DTL, FAM175A, FANCF, H2AFX, KIAA0101, KIN, MORF4L2, NPM1, NSMCE1, OGG1, PARPBP, PML, POLD2, RAD50, RAD51C, RFC4, SMARCA5, SMC4, TP53BP1, UBE2T, UBE2W,* and *WDR33;* (c) comparing the transcriptional profile for the sample of fibroblasts to a control transcriptional profile; and (d) identifying the cosmetic test agent as exhibiting potential effectiveness for providing a circadian rhythm-dependent, DNA repair benefit to the dermal layer of intrinsically-aged human skin when the transcriptional profile for the sample of fibroblasts, relative to the control transcriptional profile, corresponds to regulation of the genes in a direction indicative of said benefit. In this example, it may be desirable to generate a transcriptional profile for the test sample comprising data related to the transcription of at least two genes selected from *ATXN3, BRAC1, DTL, KIN, NPM1, PARPBP, RAD50, RAD51C, RFC4, SMC4, UBE2T,* and WDR33. In this example, it may be more desirable to use 0.1% GSE as a positive control and generate a transcriptional profile for the test sample comprising data related to the transcription of at least two genes selected from *ATXN3, BRCA1, DTL, PARPBP, RAD51C, RFC4, SMC4, UBE2T,* and WDR33. In this example, it may be even more desirable to use 0.01% GSE as a positive control and generate a transcriptional profile for the test sample comprising data related to the transcription of at least two genes selected from *KIN, NPM1, RAD50, RFC4,* and WRD33..

In some instances, it may be desirable to identify or evaluate the potential effectiveness of a cosmetic test agent for providing a circadian rhythm-dependent, DNA repair benefit to a dermal layer of photo-aged human skin. The method comprises: (a) contacting a sample of fibroblasts with a cosmetic test agent; (b) generating a transcriptional profile for the sample of fibroblasts, wherein the transcriptional profile comprises data related to the transcription of at least two genes selected from *APEX1, APEX2, APTX, CHEK1, DTL, EYA3, FAN1, FANCC, GADD45A, H2AFX, KIAA0101, MORF4L2, MSH2, NONO, NPM1, NSMCE1, PARPBP, POLD2, POLR2F, RAD51B, RAD51C, RFC4, RFC5, RPA2, SFPQ, SMC4, SWI5, TP53BP1, TP73, UBE2T, UBE2W, USP28,* and *WDR33;* (c) comparing the transcriptional profile for the sample of fibroblasts to a control transcriptional profile; and (d) identifying the cosmetic test agent as exhibiting potential effectiveness for providing a circadian rhythm-dependent, DNA repair benefit to the dermal layer of photo-aged human skin when the transcriptional profile for the sample of fibroblasts, relative to the control transcriptional profile, corresponds to regulation of the genes in a direction indicative of said benefit. In this example, it may be desirable to generate a transcriptional profile comprising data related to the transcription of at least two genes selected from *CHEK1, DTL, FAN1, GADD45A, MSH2, PARPBP, RAD51C, RFC4, RFC5, RPA2, SMC4, UBE2T,* and *WDR33.* In this example, it may be more desirable to use 0.1% GSE as a positive control and generate a transcriptional profile for the sample of fibroblasts comprising data related to the transcription of at least two genes selected from *CHEK1, DTL, FAN1, GADD45A, MSH2, PARPBP, RAD51C, RFC5, RPA2, SMC4, UBE2T,* and *WDR33.* In this example, it may be even more desirable to use 0.01% GSE as a positive control and generate a transcriptional profile for the sample of fibroblasts comprising data related to *RFC5* and *WDR33.*

In some instances, the transcriptional profile for a sample of fibroblasts contacted with a cosmetic test agent (a first sample of fibroblasts) can be compared to a second sample of fibroblasts contacted with a control agent. In such an embodiment, the cosmetic test agent can be identified as exhibiting potential effectiveness for providing a circadian rhythm-dependent, DNA repair benefit to the dermal layer of human skin when the transcriptional profiles of the first and second samples of fibroblasts, relative to one another, correspond to regulation of at least two genes of interest in a direction indicative of said benefit. The disclosed genes of interest in the above gene signatures may be suitable for use in this example. Additionally, the foregoing disclosed genes of interest may serve as indicators of previously unidentified pathways associated with DNA repair, and thus can provide opportunities for identifying new classes of cosmetic agents.

Also disclosed are methods of providing a circadian rhythm-dependent, DNA repair benefit to the skin of a subject. The methods generally describe applying a composition comprising an effective amount of Golden Silk Extract during a dark cycle of the subject's circadian cycle. In certain embodiments, the composition further comprises a dermatologically acceptable carrier. In some embodiments, the composition comprises from about 0.01% to about 0.1% by weight of Golden Silk Extract. In particular embodiments, the composition comprises about 0.01% Golden Silk Extract. In other particular embodiments, the composition comprises about 0.1% Golden Silk Extract. In some embodiments, the composition is applied for the entirety of the dark cycle of the subject's circadian cycle.

Generally, the cosmetic test agents identified by the presently-disclosed methods may be applied in accordance with cosmetic compositions and formulation parameters well-known in the art. Various methods of treatment, application, regulation, or improvement may utilize the skin care compositions comprising skin-active agents identified according to the inventive methods.

Because of the desirability of providing various cosmetic skin anti-aging benefits to a consumer, it may be beneficial to incorporate cosmetic test agents or compounds identified by one or more of the screening methods described herein into a cosmetic composition suitable for topical application to skin. That is, it may be desirable to include the cosmetic test agent as an ingredient in the cosmetic composition. In certain embodiments, the cosmetic composition may include a dermatological acceptable carrier, the test agent, and one or more optional ingredients of the kind commonly included in the particular cosmetic compositing being provided. "Dermatologically acceptable carrier" means that a carrier that is suitable for topical application to keratinous tissue, has good aesthetic properties, is compatible with the ingredients in the composition, and will not cause any unreasonable safety or toxicity concerns. Suitable carriers may include water and/or water miscible solvents. The cosmetic skin care composition may comprise from about 1% to about 95% by weight of water and/or water miscible solvent. Suitable water miscible solvents include monohydric alcohols, dihydric alcohols, polyhydric alcohols, glycerol, glycols, polyalkylene glycols such as polyethylene glycol, and mixtures thereof. When the skin care composition is in the form of an emulsion, water and/or water miscible solvents are carriers typically associated with the aqueous phase.

Suitable carriers also include oils. The skin care composition may comprise from about 1% to about 95% by weight of one or more oils. Oils may be used to solubilize, disperse, or carry materials that are not suitable for water or water soluble solvents. Suitable oils include silicones, hydrocarbons, esters, amides, ethers, and mixtures thereof. The oils may be volatile or nonvolatile.

The compositions of the present invention may contain a variety of other ingredients provided that they do not unacceptably alter the benefits of the invention. When present, compositions of the present invention may contain from about 0.0001% to about 50%; from about 0.001% to about 20%; or, alternately, from about 0.01% to about 10%, by weight of the composition, of the optional components. The amounts listed herein are only to be used as a guide, as the optimum amount of the optional components used in a composition will depend on the specific active selected since their potency does vary considerably. Hence, the amount of some optional components useful in the present invention may be outside the ranges listed herein.

The optional components, when incorporated into the composition, should be suitable for use in contact with human skin tissue without undue toxicity, incompatibility, instability, allergic response, and the like. The compositions of the present invention may include optional components such as anti-acne actives, desquamation actives, anti-cellulite agents, chelating agents, flavonoids, tanning active, non-vitamin antioxidants and radical scavengers, hair growth regulators, anti-wrinkle actives, anti-atrophy actives, minerals, phytosterols and/or plant hormones, N-acyl amino acid compounds, antimicrobial or antifungal actives, and other useful skin care actives, non-limiting examples of skin-care agents that may be suitable for use in the present composition are described in US 2006/0275237, US 2004/0175347 and The International Cosmetic Ingredient Dictionary and Handbook, Thirteenth Edition.

The skin care compositions may be generally prepared by conventional methods such as known in the art of making compositions and topical compositions. Such methods typically involve mixing of ingredients in or more steps to a relatively uniform state, with or without heating, cooling, application of vacuum, and the like. Typically, emulsions are prepared by first mixing the aqueous phase materials separately from the fatty phase materials and then combining the two phases as appropriate to yield the desired continuous phase. The compositions are preferably prepared such as to optimize stability (physical stability, chemical stability, photostability, etc.) and/or delivery of active materials.

The compositions may be in various product forms that include, but are not limited to, solutions, suspensions, lotions, creams, gels, toners, sticks, pencil, sprays, aerosols, ointments, cleansing liquid washes and solid bars, shampoos and hair conditioners, pastes, foams, powders, mousses, shaving creams, wipes, strips, patches, electrically-powered patches, wound dressing and adhesive bandages, hydrogels, film-forming products, facial and skin masks (with and without insoluble sheet), make-up such as foundations, eye liners, and eye shadows, and the like. The composition may be provided in a package sized to store a sufficient amount of the composition for a treatment period. The size, shape, and design of the package may vary widely. Certain package examples are described in U.S. Pat. Nos. D570,707; D391,162; D516,436; D535,191; D542,660; D547,193; D547,661; D558,591; D563,221; 2009/0017080; 2007/0205226; and 2007/0040306.

Non-limiting example of various aspects of the methods described herein are provided below. The examples are given solely for the purpose of illustration and are not intended to be construed as limiting the invention, as many variations thereof are possible.

### Example 1

### Testing potential cosmetic test agents

Individual experiments (referred to as batches) generally include 30 to 96 samples analyzed using Affymetrix GeneChip® technology platforms, containing 6 replicates of the vehicle control (e.g., DSMO), 2 replicate samples of a positive control that gives a strong reproducible effect in the cell type used (e.g., all trans-retinoic acid for fibroblast cells), and samples of the test material. Replication of the test material is done in separate batches due to batch effects. In vitro testing was performed in 6-well plates to provide sufficient RNA for GeneChip® analysis (2-4 µg total RNA yield/well).

Human telomerized keratinocytes (tKC) were obtained from the University of Texas, Southwestern Medical Center, Dallas, Tex. tKC cells were grown in EpiLife® media with 1× Human Keratinocyte Growth Supplement (Invitrogen, Carlsbad, Calif.) on collagen I coated cell culture flasks and plates (Becton Dickinson, Franklin Lakes, N.J.). Keratinocytes were seeded into 6-well plates at 20,000 cells/cm2 24 hours before chemical exposure. Human skin fibroblasts (BJ cell line from ATCC, Manassas, Va.) were grown in Eagle's Minimal Essential Medium (ATCC) supplemented with 10% fetal bovine serum (HyClone, Logan, Utah) in normal cell culture flasks and plates (Corning, Lowell, Mass.). BJ fibroblasts were seeded into 6-well plates at 12,000 cells/cm2 24 hours before chemical exposure.

As a non-limiting example, all cells were incubated at 37° C. in a humidified incubator with 5% CO2. At t=-24 hours cells were trypsinized from T-75 flasks and plated into 6-well plates in basal growth medium. At t=0 media was removed and replaced with the appropriate dosing solution as per the experimental design. Dosing solutions were prepared the previous day in sterile 4 ml Falcon snap cap tubes. Pure test materials may be prepared at a concentration of 1-200 µM, and botanical extracts may be prepared at a concentration of 0.001 to 1% by weight of the dosing solution. In certain embodiments, GSE (Golden Silk Extract) was used as the control test agent at concentrations from about 0.01% to about 0.1%. In specific embodiments, GSE was used at about 0.01%, while in other particular embodiments, GSE was used at about 0.1%. After 6 to 24 hours of chemical exposure, cells were viewed and imaged. The wells were examined with a microscope before cell lysis and RNA isolation to evaluate for morphologic evidence of toxicity. If morphological changes were sufficient to suggest cytotoxicity, a lower concentration of the test agent was tested. Cells were then lysed with 350 ul/well of RLT buffer containing β-mercaptoethanol (Qiagen, Valencia, Calif.), transferred to a 96-well plate, and stored at -20° C.

RNA from cell culture batches was isolated from the RLT buffer using Agencourt® RNAdvance Tissue-Bind magnetic beads (Beckman Coulter) according to manufacturer's instructions. 1 µg of total RNA per sample was labeled using Ambion Message Amp™ II Biotin Enhanced kit (Applied Biosystems Incorporated) according to manufacturer's instructions. The resultant biotin labeled and fragmented cRNA was hybridized to an Affymetrix HG-U133A 2.0 GeneChip®, which was then washed, stained and scanned using the protocol provided by Affymetrix. Alternatively, cRNA was analyzed using Affymetix HG-U219 gene arrays.

### Example 2

### Deriving a Photo-Aging Gene signature

A clinical survey study to obtain biopsy specimens for use in the investigation of gene expression patterns associated with sun light-mediated skin aging (photo-aging) was performed. Baseline gene expression patterns were examined in sun-protected and sun-exposed skin from young and aged women to examine gene expression profiles associated with photo-aging. A total of 3 full thickness skin biopsies (-4 mm) were taken from sun-protected (buttocks) and sun-exposed (extensor forearm and face) body sites from each of female volunteers (aged 20 to 74 years). There were approximately 25 subjects in each decile form 20 to 74 years old. The older women were selected to have moderate to severe forearm photo-damage. Biopsies were flash frozen in liquid nitrogen and stored at -80° C. until RNA isolation.

RNA was extracted from full thickness biopsies or from laser capture microdissected skin samples. In certain samples, the frozen skin biopsies were homogenized in Trizol (Invitrogen) and RNA extracted using the protocol provided by Invitrogen. Since the tissue samples were from full thickness biopsies, RNA was extracted from a variety of cell types within the full-thickness skin sample, including keratinocytes, fibroblasts, melanocytes, endothelial cells, pericytes, nerves, smooth muscle, sebocytes, adipocytes, and immunocytes). RNA was further purified using RNEasy spin columns (Qiagen). In other samples, laser capture microdissection was used to isolate subpopulations of cells from the tissue sections. For example, laser capture microdissection can be used to isolate the epidermal layer or dermal layer of the skin. A suitable, non-limiting example of a method of collecting biopsy samples and sectioning, sorting, and/or staining the samples in more fully described in U.S. Provisional App. No. 61/798,208 filed by Osoria, et al., on Mar. 15, 2013. In such an example, the epidermis and dermis layers of the section biopsy samples were separated with a PALM Microbeam IVTM brand Laser-capture Microdissection ("LCM") system (available from Carl Zeiss MicroImaging GmgH, Germany) in accordance with the manufacturer's instructions. Of course, it is to be appreciated that any suitable means of extracting RNA from a tissue sample known in the art may be used.

RNA was quantified using a NanoDrop spectrophotometer (Thermo Scientific, Waltham, Mass.) and quality was confirmed using an Agilent (Santa Clara, Calif.) 2100 BioAnalyzer. Total RNA was converted to GeneChip targets using the Enzo BioArray labeling procedure (Enzo Life Sciences, Farmingdale, N.Y.) and protocol provided. All biotin-labeled GeneChip targets were hybridized to Affymetrix Human Genome HG-U133 Plus 2.0 GeneChips overnight, which were then washed, stained and scanned using the protocol provided by Affymetrix. Of course, it is to be appreciated that any suitable means of quantifying and qualifying RNA from a tissue sample known in the art may be used.

The samples can be analyzed on the Affymetrix HG-U133 Plus 2.0 GeneChips, which contain 54,613 probe sets complementary to the transcripts of more than 20,000 genes. However, instances in the provided database used were derived from gene expression profiling experiments using Affymetrix HG-U133A 2.0 GeneChips, containing 22,214 probe sets, which are a subset of those present on the Plus 2.0 GeneChip. Therefore, in developing gene signatures from the clinical data, the probe sets were filtered for those included in the HG-U133A 2.0 gene chips. Alternatively, the extracted RNA was run on a GeneTitan U219 brand microarray to identify the genes that were expressed. Forearm and buttock samples were processed on the same day using the same manufacturing lot of GeneChips.

Using, generally the following selection process, a statistical analysis of the microarray data was performed to derive a plurality of photo-aging gene signatures comprising up-regulated and down-regulated genes.

### Filtering based on Absent/Margin/Present Calls.

This filter creates a list of potential genes for inclusion in the gene signature. For example, a suitable filter may be that at least 50% of the samples in one treatment group must have a Present call for each probe set. Present calls are derived from processing the raw GeneChip data and provide evidence that the gene transcript complementary to a probe set that is actually expressed in the biological sample. The probes that are absent from all samples are likely to be just noisy measurements. This step is important to filter out probe sets that do not contribute meaningful data to the signature. For both photo-aging and intrinsic aging gene signatures, the data was filtered for probe sets with at least 10% Present calls provided by the Affymetrix MAS 5 software.

### Filtering According to a Statistical Measure.

For example, a suitable statistical measure may be p-values from a t-test, ANOVA, correlation coefficient, or other model-based analysis. As one example, p-values may be chosen as the statistical measure and a cutoff value of p=0.05 may be chosen. Limiting the signature list to genes that meet some reasonable cutoff for statistical significance compared to an appropriate control is important to allow selection of genes that are characteristic of the biological state of interest. This is preferable to using a fold change value, which does not take into account the noise around the measurements. The t-statistic was used to select the probe sets in the signatures because it is signed and provides an indication of the directionality of the gene expression changes (i.e. up- or down-regulated) as well as statistical significance.

### Sorting the Probe Sets.

All the probe sets are sorted into sets of up-regulated and down-regulated sets using the statistical measure. For example, if a t-test was used to compute p-values, the values (positive and negative) of the t-statistic are used to sort the list since p-values are always positive. The sorted t-statistics will place the sets with the most significant p-values at the top and bottom of the list with the non-significant ones near the middle.

### Creation of the Gene signature.

Using the filtered and sorted list created, a suitable number of probe sets from the top and bottom are selected to create a gene signature that preferably has approximately the same number of sets chosen from the top as chosen from the bottom. For example, the gene signature created may have at least about 10, 50, 100, 200, or 300 and/or less than about 800, 600, or about 400 genes corresponding to a probe set on the chip. The number of probe sets approximately corresponds to the number of genes, but a single gene may be represented by more than one probe set. It is understood that the phrase "number of genes" as used herein, corresponds generally with the phrase "number of probe sets."

### Example 3

### Deriving an Intrinsic Aging Gene signature

A clinical survey study to obtain biopsy specimens for use in the investigation of gene expression patterns associated with chronological (intrinsic) skin aging was performed. Baseline gene expression patterns were examined in sun-protected skin from young and aged women to examine gene expression profiles associated with intrinsic aging. A total of 3 full thickness skin biopsies (-4 mm) were taken from sun-protected (buttocks) body sites from each of female volunteers (aged 20 to 74 years). There were approximately 25 subjects in each decile form 20 to 74 years old. Biopsies were flash frozen in liquid nitrogen and stored at -80° C. until RNA isolation. Using, generally the same sorting process as set forth in Example 2, RNA was extracted, purified, quantified and qualified, and analyzed. Similar to Example 2, a statistical analysis of the microarray data was performed to derive intrinsic aging gene signatures.

### Example 4

### Development of Circadian Rhythm Gene signature

A clinical survey study to obtain biopsy specimens for use in the investigation of gene expression patterns associated with establish the circadian transcriptome in healthy human skin over a 24 hour period. Biopsies were taken from 20 healthy male subjects ages 21-55, Fitzpatrick Skin Type I-III, with normal sleep patterns. Total RNA was isolated from epidermal and dermal compartments of full thickness 2 mm volar forearm punch biopsies taken at 6 hr. intervals: 12 midnight, 6 AM, 12 noon, and 6 PM. Peripheral blood and instrumental measures of the skin were also collected at 6 hr. intervals, and saliva samples collected every 3 hrs. Gene expression data presented are from epidermis only. Biopsies were flash frozen in liquid nitrogen and stored at -80° C. until RNA isolation. Using, generally the same sorting process as set forth in Example 2, RNA was extracted, purified, quantified and qualified, and analyzed. Similar to Example 2, a statistical analysis of the microarray data was performed to derive intrinsic aging gene signatures. The significance was evaluated by ANOVA model without intercept on the normalized expression value of each sample by percent change from day average.

### Example 5

### DNA Repair Theme and Network Analysis

Theme analysis was used a tool to better understand the connection and results from the photo-aging, intrinsic aging, and circadian rhythm studies and DNA-repair (DNA repair process). A suitable, non-limiting example of a method of theme analysis in more fully described in U.S. Pub. No. 20120283112 filed by Binder, et al., on Feb. 22, 2012. In short, the method uses an ontology of controlled vocabulary terms developed by the Gene Ontology (GO) Consortium that describes the biological process, molecular functions and cellular components associated with gene products. Analysis involves statistical comparison of a regulated list of genes and a larger list of all the expressed genes, to determine if genes annotated to specific GO terms are significantly enriched in the regulated list. Such analysis can be performed using Theme Extractor proprietary software and an algorithm that calculates the p value for each ontology term. In this case, the terms involved DNA repair and/or DNA repair process. Additionally, for the circadian rhythm gene signature, theme analysis can be conducted by the Database for Annotation, Visualization and Integrated Discovery on genes that are significantly differentiated to either up or down from day average at each time point (p value < .01 and .05). When multiple probe sets for a gene having less than the threshold p-value, representative single probe sets are selected by the quality of probe sets and expression value. The overrepresented biological processes were identified when False Discovery Rate (FDR) is less than 10% at some time point. For network analysis for the circadian rhythm, associations with the DNA repair process.

The various embodiments of the presently-disclosed screening methods recite various gene groupings that display a significant negative age correlation (expression becomes lower with age, p-value < 0.01), display the circadian rhythm of interest (expression is significantly elevated at midnight or early morning comparing to day average, p-value < 0.05; or significantly lower at noon comparing to day average (p-value is less than 0.05); are elevated at both midnight and early morning; and display a strong circadian pattern (p-value is less than 0.05 by one-way ANOVA test) in the circadian study)), in human skin tissue, including the epidermis and dermis layers. The sequences for the genes listed in the various groupings are the sequences listed in the GenBank® genetic sequence database housed by the National Center for Biotechnology Information ("NCBI") as of June 8, 2015. The sequences for the genes listed in the various groupings can display from about 80% to about 100% homology with the gene sequences listed in the NCBI's GenBank® database. In certain embodiments, the methods recite various gene groupings that also are significantly upregulated by GSE (about 0.01% or about 0.1%) either in 6 hour or 24 hour point in keratinocytes and/or fibroblasts.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document cited, the meaning or definition assigned to that term in this document shall govern.

## Claims

1. A screening method for evaluating or identifying the potential effectiveness of a cosmetic test agent for providing a circadian rhythm-dependent, DNA repair benefit to human skin, comprising:
a. contacting a test sample comprising skin cells with a cosmetic test agent;
b. generating a transcriptional profile for the test sample, wherein the transcriptional profile consists essentially of data related to the transcription of at least two genes selected from *APITD1, ACTR5, AP5Z1, APEX1, APEX2, APLF, APTX, ATXN3, BCCIP, BRCA1, C11orf30, CDC14B, CHEK1, CUL4A, DCLRE1C, DTL, EPC2, EXO5, EYA3, FAM175A, FAN1, FANCC, FANCF, FANCG, FANCL, FIGNL1, GADD45A, GTF2H1, H2AFX, INTS3, KIAA0430, KIAA0101, KIN, MCM9, MDC1, MGME1, MORF4L2, MRE11A, MSH2, MUM1, NEIL1, NEIL3, NONO, NPM1, NSMCE1, OGG1, PARPBP, PIF1, PML, PMS1, PMS2, POLD1, POLD2, POLE, POLK, POLR2F, RAD50, RAD51B, RAD51C, RAD52, RBM14, RECQL5, RFC4, RFC5, RPA2, SFPQ, SFR1, SMARCA5, SMC4, SMG1, SWI5, TICRR, TP53BP1, TP73, UBE2T, UBE2W, USP28, USP47, WDR33,* and *ZFYVE26*;
c. comparing the transcriptional profile for the test sample to a control transcriptional profile; and
d. identifying the cosmetic test agent as exhibiting potential effectiveness for providing a circadian rhythm-dependent, DNA repair benefit to human skin when the transcriptional profile for the test sample, relative to the control transcriptional profile, corresponds to regulation of the at least two genes in a direction indicative of said benefit.

2. The method of claim 1, wherein the circadian rhythm-dependent, DNA repair benefit is for photo-aged human skin, and the transcriptional profile consists essentially of data related to the transcription of at least two genes selected from *APEX1, CHEK1, DTL, EYA3, FAN1, H2AFX, MORF4L2, MSH2, NONO, NSMCE1, POLD2, RAD51C, RFC4, SWI5, TP53BP1, UBE2T, UBE2W,* and *WDR33.*

3. The method of claim 1, wherein the circadian rhythm-dependent, DNA repair benefit is for intrinsically-aged human skin, and the transcriptional profile consists essentially of data related to the transcription of at least two genes selected from *APITD1, APEX1, APTX, ATXN3, BRCA1, C11orf30, CDC14B, CHEK1, DCLRE1C, DTL, EPC2, EXO5, EYA3, FAM175A, FANCF, GADD45A, GTF2H1, H2AFX, KIAA0101, KIN, MORF4L2, NEIL3, NPM1, NSMCE1, OGG1, PARPBP, PIF1, PML, POLD2, RAD50, RAD51B, RAD51C, RFC4, SMARCA5, SMC4, TP53BP1, UBE2T, UBE2W,* and *WDR33.*

4. The method of claim 1, wherein the test sample is selected from full-thickness human skin tissue, keratinocytes, and fibroblasts.

5. The method of claim 4, wherein the circadian rhythm-dependent, DNA repair benefit is for an epidermal layer of human skin, the test sample is keratinocytes, and the transcriptional profile consists essentially of data related to the transcription of at least two genes selected from *APITD1, APEX1, APTX, ATXN3, BRCA1, C11orf30, CDC14B, CHEK1, CUL4A, DCLRE1C, DTL, EPC2, EYA3, EXO5, FAM175A, FAN1, FANCF, GADD45A, GTF2H1, H2AFX, KIAA0101, KIN, MORF4L2, NEIL3, NONO, NPM1, NSMCE1, OGG1, PARPBP, PIF1, PML, PMS2, POLD2, RAD50, RAD51B, RAD51C, RFC4, SMARCA5, SMC4, TP53BP1, UBE2T, UBE2W,* and *WDR33.*

6. The method of claim 5, wherein the circadian rhythm-dependent, DNA repair benefit is for an epidermal layer of photo-aged human skin, and the transcriptional profile consists essentially of data related to the transcription of at least two genes selected from *CUL4A, EYA3, FAN1, NONO, OGG1, and PMS2.*

7. The method of claim 5, wherein the circadian rhythm-dependent, DNA repair benefit is for an epidermal layer of intrinsically-aged human skin, and the transcriptional profile consists essentially of data related to the transcription of at least two genes selected from *APITD1, APEX1, APTX, ATXN3, BRCA1, C11orf30, CDC14B, CHEK1, DCLRE1C, DTL, EPC2, EXO5, EYA3, FAM175A, FANCF, GADD45A, GTF2H1, H2AFX, KIAA0101, KIN, MORF4L2, NEIL3, NPM1, NSMCE1, OGG1, PARPBP, PIF1, PML, POLD2, RAD50, RAD51B, RAD51C, RFC4, SMARCA5, SMC4, TP53BP1, UBE2T, UBE2W,* and *WDR33.*

8. The method of claim 4, wherein the circadian rhythm-dependent, DNA repair benefit is for a dermal layer of human skin, the test sample is fibroblasts, and the transcriptional profile consists essentially of data related to the transcription of at least two genes selected from *APEX1, APEX2, APTX, ATXN3, BRCA1, C11orf30, CHEK1, DTL, EYA3, FAM175A, FAN1, FANCC, FANCF, GADD45A, H2AFX, KIAA0101, KIN, MORF4L2, MSH2, NONO, NPM1, NSMCE1, OGG1, PARPBP, PML, POLD2, POLR2F, RAD50, RAD51B, RAD51C, RFC4, RFC5, RPA2, SFPQ, SMC4, SMARCA5, SWI5, TP53BP1, TP73, UBE2T, UBE2W, USP28,* and *WDR33.*

9. The method of claim 8, wherein the circadian rhythm-dependent, DNA repair benefit is for a dermal layer of photo-aged human skin, and the transcriptional profile consists essentially of data related to the transcription of at least two genes selected from *APEX1, APEX2, APTX, CHEK1, DTL, EYA3, FAN1, FANCC, GADD45A, H2AFX, KIAA0101, MORF4L2, MSH2, NONO, NPM1, NSMCE1, PARPBP, POLD2, POLR2F, RAD51B, RAD51C, RFC4, RFC5, RPA2, SFPQ, SMC4, SWI5, TP53BP1, TP73, UBE2T, UBE2W, USP28,* and *WDR33.*

10. The method of claim 8, wherein the circadian rhythm-dependent, DNA repair benefit is for a dermal layer of intrinsically-aged human skin, and the transcriptional profile consists essentially of data related to the transcription of at least two genes selected from *APEX1, APTX, ATXN3, BRCA1, C11orf30, DTL, FAM175A, FANCF, H2AFX, KIAA0101, KIN, MORF4L2, NPM1, NSMCE1, 0001, PARPBP, PML, POLD2, RAD50, RAD51C, RFC4, SMARCA5, SMC4, TP53BP1, UBE2T, UBE2W,* and *WDR33.*

11. The method of any one of claims 1 to 10, wherein the control profile is generated by contacting a second test sample with a positive control and the transcriptional profile and the control profile are concordant.

12. The method of claim 11, wherein the test sample is full-thickness human skin tissue subjected to laser capture microdissection.

13. The method of claim 12, wherein human skin tissue is separated into an epidermal layer and a dermal layer by the laser capture microdissection.

14. The method of any one of claims 1 to 13, further comprising: isolating RNA from the test sample, using the isolated RNA to create cRNA, labeling the cRNA, and hybridizing the labeled cRNA to a microarray.

## Patentansprüche

1. Screening-Verfahren zum Bewerten oder Identifizieren der potentiellen Wirksamkeit eines kosmetischen Testmittels zum Bereitstellen einer biorhythmusabhängigen DNA-Reparatur-Nutzwirkung an menschliche Haut, umfassend:
a. Inkontaktbringen einer Testprobe, umfassend Hautzellen, mit einem kosmetischen Testmittel;
b. Generieren eines Transkriptionsprofils für die Testprobe, wobei das Transkriptionsprofil im Wesentlichen aus Daten besteht, die die Transkription von mindestens zwei Genen betreffen, ausgewählt aus *APITD1, ACTR5, AP5Z1, APEX1, APEX2, APLF, APTX, ATXN3, BCCIP, BRCA1, C11orf30, CDC14B, CHEK1, CUL4A, DCLRE1C, DTL, EPC2, EXO5, EYA3, FAM175A, FAN1, FANCC, FANCF, FANCG, FANCL, FIGNL1, GADD45A, GTF2H1, H2AFX, INTS3, KIAA0430, KIAA0101, KIN, MCM9, MDC1, MGME1, MORF4L2, MRE11A, MSH2, MUM1, NEIL1, NEIL3, NONO, NPM1, NSMCE1, OGG1, PARPBP, PIF1, PML, PMS1, PMS2, POLD1, POLD2, POLE, POLK, POLR2F, RAD50, RAD51B, RAD51C, RAD52, RBM14, RECQL5, RFC4, RFC5, RPA2, SFPQ, SFR1, SMARCA5, SMC4, SMG1, SWI5, TICRR, TP53BP1, TP73, UBE2T, UBE2W, USP28, USP47, WDR33* und *ZFYVE26;*
c. Vergleichen des Transkriptionsprofils für die Testprobe mit einem Kontroll-Transkriptionsprofil; und
d. Identifizieren des kosmetischen Testmittels als potentiell Wirksamkeit für das Bereitstellen einer biorhythmusabhängigen DNA-Reparatur-Nutzwirkung an menschliche Haut vorweisend, wenn das Transkriptionsprofil für die Testprobe, bezogen auf das Kontroll-Transkriptionsprofil, einer Regulierung der mindestens zwei Gene in eine Richtung entspricht, die auf die Nutzwirkung hindeutet.

2. Verfahren nach Anspruch 1, wobei die biorhythmusabhängige DNA-Reparatur-Nutzwirkung für lichtgealterte menschliche Haut ist, und das Transkriptionsprofil im Wesentlichen aus Daten besteht, die die Transkription von mindestens zwei Genen betreffen, ausgewählt aus *APEX1, CHEK1, DTL, EYA3, FAN1, H2AFX, MORF4L2, MSH2, NONO, NSMCE1, POLD2, RAD51C, RFC4, SWI5, TP53BP1, UBE2T, UBE2W und WDR33.*

3. Verfahren nach Anspruch 1, wobei die biorhythmusabhängige DNA-Reparatur-Nutzwirkung für intrinsisch gealterte menschliche Haut ist, und das Transkriptionsprofil im Wesentlichen aus Daten besteht, die die Transkription von mindestens zwei Genen betreffen, ausgewählt aus *APITD1, APEX1, APTX, ATXN3, BRCA1, C11orf30, CDC14B, CHEK1, DCLRE1C, DTL, EPC2, EXO5, EYA3, FAM175A, FANCF, GADD45A, GTF2H1, H2AFX, KIAA0101, KIN, MORF4L2, NEIL3, NPM1, NSMCE1, OGG1, PARPBP, PIF1, PML, POLD2, RAD50, RAD51B, RAD51C, RFC4, SMARCA5, SMC4, TP53BP1, UBE2T, UBE2W und WDR33.*

4. Verfahren nach Anspruch 1, wobei die Testprobe ausgewählt ist aus menschlichem Hautgewebe voller Dicke, Keratinozyten und Fibroblasten.

5. Verfahren nach Anspruch 4, wobei die biorhythmusabhängige DNA-Reparatur-Nutzwirkung für eine epidermale Schicht von menschlicher Haut ist, die Testprobe Keratinozyten ist, und das Transkriptionsprofil im Wesentlichen aus Daten besteht, die die Transkription von mindestens zwei Genen betreffen, ausgewählt aus *APITD1, APEX1, APTX, ATXN3, BRCA1, C11orf30, CDC14B, CHEK1, CUL4A, DCLRE1C, DTL, EPC2, EYA3, EXO5, FAM175A, FAN1, FANCF, GADD45A, GTF2H1, H2AFX, KIAA0101, KIN, MORF4L2, NEIL3, NONO, NPM1, NSMCE1, OGG1, PARPBP, PIF1, PML, PMS2, POLD2, RAD50, RAD51B, RAD51C, RFC4, SMARCA5, SMC4, TP53BP1, UBE2T, UBE2W* und *WDR33.*

6. Verfahren nach Anspruch 5, wobei die biorhythmusabhängige DNA-Reparatur-Nutzwirkung für eine epidermale Schicht von lichtgealterter menschlicher Haut ist, und das Transkriptionsprofil im Wesentlichen aus Daten besteht, die die Transkription von mindestens zwei Genen betreffen, ausgewählt aus *CUL4A, EYA3, FAN1, NONO, OGG1* und *PMS2.*

7. Verfahren nach Anspruch 5, wobei die biorhythmusabhängige DNA-Reparatur-Nutzwirkung für eine epidermale Schicht von intrinsisch gealterter menschlicher Haut ist, und das Transkriptionsprofil im Wesentlichen aus Daten besteht, die die Transkription von mindestens zwei Genen betreffen, ausgewählt aus *APITD1, APEX1, APTX, ATXN3, BRCA1, C11orf30, COC14B, CHEK1, DCLRE1C, DTL, EPC2, EXO5, EYA3, FAM175A, FANCF, GADD45A, GTF2H1, H2AFX, KIAA0101, KIN, MORF4L2, NEIL3, NPM1, NSMCE1, OGG1, PARPBP, PIF1, PML, POLD2, RAD50, RAD51B, RAD51C, RFC4, SMARCA5, SMC4, TP53BP1, UBE2T, UBE2W* und *WDR33.*

8. Verfahren nach Anspruch 4, wobei die biorhythmusabhängige DNA-Reparatur-Nutzwirkung für eine dermale Schicht von menschlicher Haut ist, die Testprobe Fibroblasten ist, und das Transkriptionsprofil im Wesentlichen aus Daten besteht, die die Transkription von mindestens zwei Genen betreffen, ausgewählt aus *APEX1, APEX2, APTX, ATXN3, BRCA1, C11orf30, CHEK1, DTL, EYA3, FAM175A, FAN1, FANCC, FANCF, GADD45A, H2AFX, KIAA0101, KIN, MORF4L2, MSH2, NONO, NPM1, NSMCE1, OGG1, PARPBP, PML, POLD2, POLR2F, RAD50, RAD51B, RAD51C, RFC4, RFC5, RPA2, SFPQ, SMC4, SMARCA5, SWI5, TP53BP1, TP73, UBE2T, UBE2W, USP28* und *WDR33.*

9. Verfahren nach Anspruch 8, wobei die biorhythmusabhängige DNA-Reparatur-Nutzwirkung für eine dermale Schicht von lichtgealterter menschlicher Haut ist, und das Transkriptionsprofil im Wesentlichen aus Daten besteht, die die Transkription von mindestens zwei Genen betreffen, ausgewählt aus *APEX1, APEX2, APTX, CHEK1, DTL, EYA3, FAN1, FANCC, GADD45A, H2AFX, KIAA0101, MORF4L2, MSH2, NONO, NPM1, NSMCE1, PARPBP, POLD2, POLR2F, RAD51B, RAD51C, RFC4, RFC5, RPA2, SFPQ, SMC4, SWI5, TP53BP1, TP73, UBE2T, UBE2W, USP28* und *WDR33.*

10. Verfahren nach Anspruch 8, wobei die biorhythmusabhängige DNA-Reparatur-Nutzwirkung für eine dermale Schicht von intrinsisch gealterter menschlicher Haut ist, und das Transkriptionsprofil im Wesentlichen aus Daten besteht, die die Transkription von mindestens zwei Genen betreffen, ausgewählt aus *APEX1, APTX, ATXN3, BRCA1, C11orf30, DTL, FAM175A, FANCF, H2AFX, KIAA0101, KIN, MORF4L2, NPM1, NSMCE1, OGG1, PARPBP, PML, POLD2, RAD50, RAD51C, RFC4, SMARCA5, SMC4, TP53BP1, UBE2T, UBE2W* und *WDR33.*

11. Verfahren nach einem der Ansprüche 1 bis 10, wobei das Kontrollprofil durch Inkontaktbringen einer zweiten Testprobe mit einer Positivkontrolle generiert wird, und das Transkriptionsprofil und das Kontrollprofil übereinstimmend sind.

12. Verfahren nach Anspruch 11, wobei die Testprobe menschliches Hautgewebe voller Dicke ist, das einer Lasermikrodissektion unterzogen wird.

13. Verfahren nach Anspruch 12, wobei menschliches Hautgewebe durch die Lasermikrodissektion in eine epidermale Schicht und eine dermale Schicht getrennt wird.

14. Verfahren nach einem der Ansprüche 1 bis 13, ferner umfassend: Isolieren von RNA aus der Testprobe, Verwenden der isolierten RNA, um cRNA zu erzeugen, Markieren der cRNA und Hybridisieren der markierten cRNA an ein Mikroarray.

## Revendications

1. Procédé de criblage pour évaluer ou identifier l'efficacité potentielle d'un agent de test cosmétique pour fournir un effet bénéfique de réparation d'ADN, dépendant du rythme circadien à la peau humaine, comprenant :
a. la mise en contact d'un échantillon de test comprenant des cellules de peau avec un agent de test cosmétique ;
b. la génération d'un profil transcriptionnel pour l'échantillon de test, dans lequel le profil transcriptionnel est constitué sensiblement de données se rapportant à la transcription d'au moins deux gènes choisis parmi *APITD1, ACTR5, AP5Z1, APEX1, APEX2, APLF, APTX, ATXN3, BCCIP, BRCA1, C11orf30, CDC14B, CHEK1, CUL4A, OCLRE1C, DTL, EPC2, EXO5, EYA3, FAM175A, FAN1, FANCC, FANCF, FANCG, FANCL, FIGNL1, GADD45A, GTF2H1, H2AFX, INTS3, KIAA0430, KIAA0101, KIN, MCM9, MDC1, MGME1, MORF4L2, MRE11A, MSH2, MUM1, NEIL1, NEIL3, NONO, NPM1, NSMCE1, OGG1, PARPBP, PIF1, PML, PMS1, PMS2, POLD1, POLD2, POLE, POLK, POLR2F, RAD50, RAD51B, RAD51C, RAD52, RBM14, RECQL5, RFC4, RFC5, RPA2, SFPQ, SFR1, SMARCA5, SMC4, SMG1, SWI5, TICRR, TP53BP1, TP73, UBE2T, UBE2W, USP28, USP47, WDR33* et *ZFYVE26 ;*
c. la comparaison du profil transcriptionnel pour l'échantillon de test à un profil transcriptionnel témoin ; et
d. l'identification de l'agent de test cosmétique comme présentant une efficacité potentielle pour fournir un effet bénéfique de réparation d'ADN, dépendant du rythme circadien à la peau humaine lorsque le profil transcriptionnel pour l'échantillon de test, par rapport au profil transcriptionnel témoin, correspond à une régulation des au moins deux gènes dans une direction caractéristique dudit effet bénéfique.

2. Procédé selon la revendication 1, dans lequel l'effet bénéfique de réparation d'ADN, dépendant du rythme circadien est pour une peau humaine vieillie par rayonnement, et le profil transcriptionnel est constitué sensiblement de données se rapportant à la transcription d'au moins deux gènes choisis parmi *APEX1, CHEK1, DTL, EYA3, FAN1, H2AFX, MORF4L2, MSH2, NONO, NSMCE1, POLD2, RAD51C, RFC4, SWI5, TP53BP1, UBE2T, UBE2W* et *WDR33.*

3. Procédé selon la revendication 1, dans lequel l'effet bénéfique de réparation d'ADN, dépendant du rythme circadien est pour une peau humaine intrinsèquement vieillie, et le profil transcriptionnel est constitué sensiblement de données se rapportant à la transcription d'au moins deux gènes choisis parmi *APITD1, APEX1, APTX, ATXN3, BRCA1, C11orf30, CDC14B, CHEK1, DCLRE1C, DTL, EPC2, EXO5, EYA3, FAM175A, FANCF, GADD45A, GTF2H1, H2AFX, KIAA0101, KIN, MORF4L2, NEIL3, NPM1, NSMCE1, OGG1, PARPBP, PIF1, PML, POLD2, RAD50, RAD51B, RAD51C, RFC4, SMARCA5, SMC4, TP53BP1, UBE2T, UBE2W* et *WDR33.*

4. Procédé selon la revendication 1, dans lequel l'échantillon de test est choisi parmi un tissu cutané humain sur toute son épaisseur, des kératinocytes et des fibroblastes.

5. Procédé selon la revendication 4, dans lequel l'effet bénéfique de réparation d'ADN, dépendant du rythme circadien est pour une couche épidermique de peau humaine, l'échantillon de test est des kératinocytes, et le profil transcriptionnel est constitué sensiblement de données se rapportant à la transcription d'au moins deux gènes choisis parmi *APITD1, APEX1, APTX, ATXN3, BRCA1, C11orf30, CDC14B, CHEK1, CUL4A, DCLRE1C, DTL, EPC2, EYA3, EXO5, FAM175A, FAN1, FANCF, GADD45A, GTF2H1, H2AFX, KIAA0101, KIN, MORF4L2, NEIL3, NONO, NPM1, NSMCE1, OGG1, PARPBP, PIF1, PML, PMS2, POLD2, RAD50, RAD51B, RAD51C, RFC4, SMARCA5, SMC4, TP53BP1, UBE2T, UBE2W* et *WDR33.*

6. Procédé selon la revendication 5, dans lequel l'effet bénéfique de réparation d'ADN, dépendant du rythme circadien est pour une couche épidermique de peau humaine vieillie par rayonnement, et le profil transcriptionnel est constitué sensiblement de données se rapportant à la transcription d'au moins deux gènes choisis parmi *CUL4A, EYA3, FAN1, NONO, OGG1* et *PMS2.*

7. Procédé selon la revendication 5, dans lequel l'effet bénéfique de réparation d'ADN, dépendant du rythme circadien est pour une couche épidermique de peau humaine intrinsèquement vieillie, et le profil transcriptionnel est constitué sensiblement de données se rapportant à la transcription d'au moins deux gènes choisis parmi *APITD1, APEX1, APTX, ATXN3, BRCA1, C11orf30, CDC14B, CHEK1, DCLRE1C, DTL, EPC2, EXO5, EYA3, FAM175A, FANCF, GADD45A, GTF2H1, H2AFX, KIAA0101, KIN, MORF4L2, NEIL3, NPM1, NSMCE1, OGG1, PARPBP, PIF1, PML, POLD2, RAD50, RAD51B, RAD51C, RFC4, SMARCA5, SMC4, TP53BP1, UBE2T, UBE2W* et *WDR33.*

8. Procédé selon la revendication 4, dans lequel l'effet bénéfique de réparation d'ADN, dépendant du rythme circadien est pour une couche dermique de peau humaine, l'échantillon de test est des fibroblastes, et le profil transcriptionnel est constitué sensiblement de données se rapportant à la transcription d'au moins deux gènes choisis parmi *APEX1, APEX2, APTX, ATXN3, BRCA1, C11orf30, CHEK1, DTL, EYA3, FAM175A, FAN1, FANCC, FANCF, GADD45A, H2AFX, KIAA0101, KIN, MORF4L2, MSH2, NONO, NPM1, NSMCE1, OGG1, PARPBP, PML, POLD2, POLR2F, RAD50, RAD51B, RAD51C, RFC4, RFC5, RPA2, SFPQ, SMC4, SMARCA5, SWI5, TP53BP1, TP73, UBE2T, UBE2W, USP28* et *WDR33.*

9. Procédé selon la revendication 8, dans lequel l'effet bénéfique de réparation d'ADN, dépendant du rythme circadien est pour une couche dermique de peau humaine vieillie par rayonnement, et le profil transcriptionnel est constitué sensiblement de données se rapportant à la transcription d'au moins deux gènes choisis parmi *APEX1, APEX2, APTX, CHEK1, DTL, EYA3, FAN1, FANCC, GADD45A, H2AFX, KIAA0101, MORF4L2, MSH2, NONO, NPM1, NSMCE1, PARPBP, POLD2, POLR2F, RAD51B, RAD51C, RFC4, RFC5, RPA2, SFPQ, SMC4, SWI5, TP53BP1, TP73, UBE2T, UBE2W, USP28* et *WDR33.*

10. Procédé selon la revendication 8, dans lequel l'effet bénéfique de réparation d'ADN, dépendant du rythme circadien est pour une couche dermique de peau humaine intrinsèquement vieillie, et le profil transcriptionnel est constitué sensiblement de données se rapportant à la transcription d'au moins deux gènes choisis parmi *APEX1, APTX, ATXN3, BRCA1, C11orf30, DTL, FAM175A, FANCF, H2AFX, KIAA0101, KIN, MORF4L2, NPM1, NSMCE1, OGG1, PARPBP, PML, POLD2, RAD50, RAD51C, RFC4, SMARCA5, SMC4, TP53BP1, UBE2T, UBE2W* et *WDR33.*

11. Procédé selon l'une quelconque des revendications 1 à 10, dans lequel le profil témoin est généré par mise en contact d'un deuxième échantillon de test avec un témoin positif, et le profil transcriptionnel et le profil témoin sont concordants.

12. Procédé selon la revendication 11, dans lequel l'échantillon de test est un tissu cutané humain sur toute son épaisseur soumis à une microdissection par capture au laser.

13. Procédé selon la revendication 12, dans lequel le tissu cutané humain est séparé en une couche épidermique et une couche dermique par la microdissection par capture au laser.

14. Procédé selon l'une quelconque des revendications 1 à 13, comprenant en outre : l'isolement d'ARN de l'échantillon de test, l'utilisation de l'ARN isolé pour créer de l'ARNc, le marquage de l'ARNc, et l'hybridation de l'ARNc marqué à une micromatrice.
